# EUROPEAN PATENT SPECIFICATION

(11) **EP 4 389 743 B1**
(45) Date of publication and mention of the grant of the patent: **27.08.2025**
(21) Application number: 22214548.4
(22) Date of filing: 19.12.2022
(51) Int. Cl.: C07D 403/10, H10K 50/18, H10K 85/60

(54) **COMPOUND, SEMICONDUCTING MATERIAL, ORGANIC ELECTRONIC DEVICE, DISPLAY DEVICE AND METHOD FOR PREPARING THE SAME**
VERBINDUNG, HALBLEITERMATERIAL, ORGANISCHE ELEKTRONISCHE VORRICHTUNG, ANZEIGEVORRICHTUNG UND VERFAHREN ZUR HERSTELLUNG DAVON
COMPOSÉ, MATÉRIAU SEMI-CONDUCTEUR, DISPOSITIF ÉLECTRONIQUE ORGANIQUE, DISPOSITIF D'AFFICHAGE ET SON PROCÉDÉ DE PRÉPARATION

(43) Date of publication of application: 26.06.2024
(73) Proprietor: Novaled GmbH, 01099 Dresden (DE)
(72) Inventor: GALÁN GARÍA, Elena, 01099 Dresden (DE); PAVICIC, Domagoj, 01099 Dresden (DE); KARPOV, Yevhen, 01099 Dresden (DE); STENNETT, Thomas, 01099 Dresden (DE)
(74) Representative: Bittner, Thomas L.

(56) References cited:
- WO-A1-2019/208991
- WO-A1-2022/015047
- WO-A1-2023/025839

## Description

### TECHNICAL FIELD

The present invention relates to a compound. The present invention relates further to a semiconducting material comprising the compound, to an organic electronic device comprising the semiconducting material, to a display device comprising the organic electronic device, and to a process for preparing the organic electronic device.

### BACKGROUND OF THE INVENTION

Organic semiconducting devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing electrochemical stability is needed so that the organic semiconducting device, such as an organic light emitting diode, may be applied to a large-size flat panel display.

Further, development of an organic semiconductor layer being capable to have an extended life span at higher current density and thereby at higher brightness is needed. In particular, the development of an organic semiconductor material or semiconductor layer is needed with respect to lowering the operating voltage, which is important for reducing power consumption and increasing battery life, for example of a mobile display device.

It is, therefore, the object of the present invention to provide compounds and semiconducting materials for preparing organic electronic devices and display devices overcoming drawbacks of the prior art, in particular with improved performance, especially with improved lifetime and current efficiency.

### DISCLOSURE

This object is achieved by a compound of formula (I) 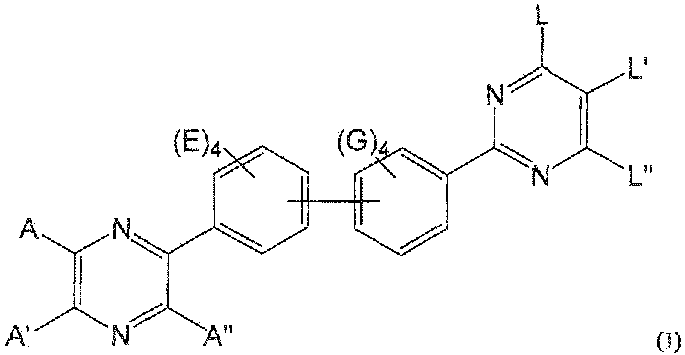 wherein
- A, A' and A" are independently selected from the group consisting of substituted or unsubstituted C₁ to C₂₀ alkyl, substituted or unsubstituted C₁ to C₂₀ alkenyl, substituted or unsubstituted C₆ to C₆₀ aryl, and substituted or unsubstituted C₂ to C₆₀ heteroaryl;
- L and L" are independently selected from the group consisting of substituted or unsubstituted C₆ to C₆₀ aryl and substituted or unsubstituted C₂ to C₆₀ heteroaryl, and L' is selected from the group consisting of H, D, C₁ to C₂₀ alkyl, substituted or unsubstituted C₁ to C₂₀ alkenyl, substituted or unsubstituted C₆ to C₆₀ aryl, and substituted or unsubstituted C₂ to C₆₀ heteroaryl;
   or
- L is selected from the group consisting of substituted or unsubstituted C₆ to C₆₀ aryl and substituted and unsubstituted C₂ to C₆₀ heteroaryl, and L' and L" form together a condensed 6-membered aromatic ring,
wherein the condensed 6-membered aromatic ring
- is unsubstituted,
   or
- is substituted with one or more substituent(s) independently selected from (substituted or unsubstituted) C₆ to C₅₆ aryl, provided that the total number of C-atoms of all substituents does not exceed 56,
   or
- is condensed with a second 6-membered aromatic ring, wherein the condensed 6-membered aromatic ring and the second 6-membered aromatic ring are unsubstituted or at least one of the condensed 6-membered aromatic ring and the second 6-membered aromatic ring is substituted with one or more substituent(s) independently selected from (substituted or unsubstituted) C₆ to C₄₀ aryl provide that the total number of C-atoms of all substituents does not exceed 40;

- E is independently selected from the group consisting of H, D, halogen, SiR^{a}R^{b}R^{c} (wherein R^{a}, R^{b} and R^{c} are independently selected from the group consisting of C₁ to C₆ alkyl and phenyl), C₁ to C₆ alkyl, C₁ to C₆ alkoxy, CN and P(=O)R^{d}R^{e} (wherein R^{d} and R^{e} are independently selected from the group consisting of C₁ to C₆ alkyl and phenyl);
- G is independently selected from the group consisting of H, D, halogen, SiR^{a}R^{b}R^{c} (wherein R^{a}, R^{b} and R^{c} are independently selected from the group consisting of C₁ to C₆ alkyl and phenyl), C₁ to C₆ alkyl, C₁ to C₆ alkoxy, CN and P(=O)R^{d}R^{e} (wherein R^{d} and R^{e} are independently selected from the group consisting of C₁ to C₆ alkyl and phenyl).

This object is further achieved by a semiconducting material comprising the compound of formula (I) according to the present invention.

This object is further achieved by an organic electronic device comprising a semiconducting layer, wherein the semiconducting layer comprises the semiconducting material according to the present invention.

This object is further achieved by a display device comprising the organic electronic device according to the present invention.

This object is further achieved by a process for preparing the organic electronic device according to the invention, wherein the process comprises a step of depositing the compound according to the present invention on a solid support.

Surprisingly, it was found that the compound according to the invention, respectively the semiconducting material comprising the same, can be used for preparing organic electronic devices and display devices having improved properties in comparison with respective devices of the prior art, especially with improved lifetime and current efficiency.

### Compound

According to one aspect, the invention is related to a compound of formula (I)

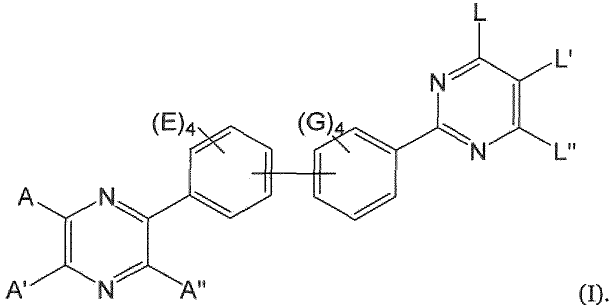

If not mentioned else explicitly, all compounds, groups, moieties, substituents etc. shown herein, especially by structural formulas, by systematic names etc. encompass the respective partially and fully deuterated derivatives thereof.

In accordance with the present disclosure, in a formula showing the following binding situation, 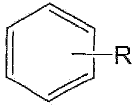 the group R may be bound to any suitable binding position.

For example, the biphenylene spacer 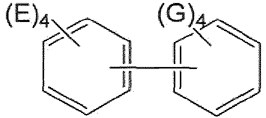 may be a 1,1'-biphenyl-4,4'-diyl; 1,1'-biphenyl-3,4'-diyl; 1,1'-biphenyl-2,4'-diyl; 1,1'-biphenyl-3,3'-diyl; 1,1'-biphenyl-2,3'-diyl; 1,1'-biphenyl-2,2'-diyl.

In a specific embodiment, the biphenylene spacer may have one of the following structures 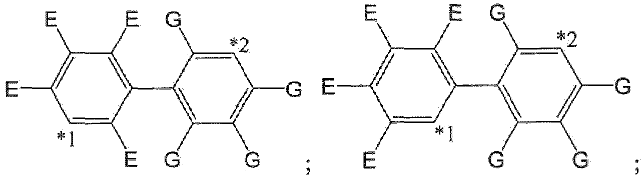 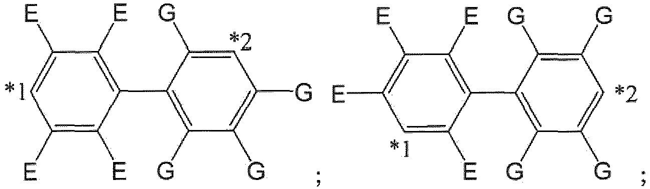 wherein the biphenylene spacer is bonded at *1 to 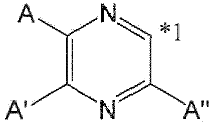 and the biphenylene spacer is bonded at *2 to 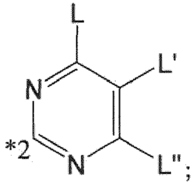 and E and G are independently selected from the group consisting of H, D, halogen, SiR^{a}R^{b}R^{c} (wherein R^{a}, R^{b} and R^{c} are independently selected from the group consisting of C₁ to C₆ alkyl and phenyl), C₁ to C₆ alkyl, C₁ to C₆ alkoxy, CN and P(=O)R^{d}R^{e} (wherein R^{d} and R^{e} are independently selected from the group consisting of C₁ to C₆ alkyl and phenyl).

A, A' and A" are independently selected from the group consisting of substituted or unsubstituted C₁ to C₂₀ alkyl, substituted or unsubstituted C₁ to C₂₀ alkenyl, substituted or unsubstituted C₆ to C₆₀ aryl, and substituted or unsubstituted C₂ to C₆₀ heteroaryl.

The one or more substituent(s) on A, A' and A" in case that the respective group is substituted are independently selected from the group consisting of D, halogen, SiR^{a}R^{b}R^{c} (wherein R^{a}, R^{b} and R^{c} are independently selected from the group consisting of C₁ to C₆ alkyl and phenyl), C₁ to C₆ alkyl, C₁ to C₆ alkoxy, CN and P(=O)R^{d}R^{e} (wherein R^{d} and R^{e} are independently selected from the group consisting of C₁ to C₆ alkyl and phenyl).

A, A' and A" may be independently selected from the group consisting of substituted or unsubstituted C₁ to C₁₂ alkyl, substituted or unsubstituted C₁ to C₁₂ alkenyl, substituted or unsubstituted C₆ to C₄₈ aryl, and substituted or unsubstituted C₂ to C₄₇ heteroaryl. A, A' and A" may be independently selected from the group consisting of substituted or unsubstituted C₁ to C₆ alkyl, substituted or unsubstituted C₁ to C₆ alkenyl, substituted or unsubstituted C₆ to C₃₆ aryl, and substituted or unsubstituted C₂ to C₃₆ heteroaryl. A, A' and A" may be independently selected from the group consisting of substituted or unsubstituted C₁ to C₄ alkyl, substituted or unsubstituted C₁ to C₄ alkenyl, substituted or unsubstituted C₆ to C₁₈ aryl, and substituted or unsubstituted C₂ to C₁₈ heteroaryl. A, A' and A" may be independently selected from the group consisting of substituted or unsubstituted C₁ to C₄ alkyl, substituted or unsubstituted C₁ to C₄ alkenyl, substituted or unsubstituted phenyl, and substituted or unsubstituted C₂ to C₅ heteroaryl.

A, A' and A" may be independently selected from the group consisting of substituted or unsubstituted C₆ to C₆₀ aryl, and substituted or unsubstituted C₂ to C₆₀ heteroaryl. A, A' and A" may be independently selected from the group consisting of substituted or unsubstituted C₆ to C₄₈ aryl, and substituted or unsubstituted C₂ to C₄₇ heteroaryl. A, A' and A" may be independently selected from the group consisting of substituted or unsubstituted C₆ to C₃₆ aryl, and substituted or unsubstituted C₂ to C₃₆ heteroaryl. A, A' and A" may be independently selected from the group consisting of substituted or unsubstituted C₆ to C₁₈ aryl, and substituted or unsubstituted C₂ to C₁₈ heteroaryl. A, A' and A" may be independently selected from the group consisting of substituted or unsubstituted phenyl, and substituted or unsubstituted C₂ to C₅ heteroaryl.

A, A' and A" may be independently selected from the group consisting of substituted or unsubstituted C₆ to C₆₀ aryl. A, A' and A" may be independently selected from the group consisting of substituted or unsubstituted C₆ to C₄₈ aryl. A, A' and A" may be independently selected from the group consisting of substituted or unsubstituted C₆ to C₃₆ aryl. A, A' and A" may be independently selected from the group consisting of substituted or unsubstituted C₆ to C₁₈ aryl. A, A' and A" may be independently selected from the group consisting of substituted or unsubstituted phenyl. A, A' and A" may be each unsubstituted phenyl.

In a first alternative, L and L" are independently selected from the group consisting of substituted or unsubstituted C₆ to C₆₀ aryl and substituted or unsubstituted C₂ to C₆₀ heteroaryl, and L' is selected from the group consisting of H, D, C₁ to C₂₀ alkyl, substituted or unsubstituted C₁ to C₂₀ alkenyl, substituted or unsubstituted C₆ to C₆₀ aryl, and substituted or unsubstituted C₂ to C₆₀ heteroaryl.

The one or more substituent(s) on L, L' and L" in case that the respective group is substituted are independently selected from the group consisting of D, halogen, SiR^{a}R^{b}R^{c} (wherein R^{a}, R^{b} and R^{c} are independently selected from the group consisting of C₁ to C₆ alkyl and phenyl), C₁ to C₆ alkyl, C₁ to C₆ alkoxy, CN and P(=O)R^{d}R^{e} (wherein R^{d} and R^{e} are independently selected from the group consisting of C₁ to C₆ alkyl and phenyl).

In this first alternative, L and L" may be independently selected from the group consisting of substituted or unsubstituted C₆ to C₄₈ aryl and substituted or unsubstituted C₂ to C₄₇ heteroaryl, and L' may be selected from the group consisting of H, D, C₁ to C₁₂ alkyl, substituted or unsubstituted C₁ to C₁₂ alkenyl, substituted or unsubstituted C₆ to C₄₈ aryl, and substituted or unsubstituted C₂ to C₄₇ heteroaryl. In this first alternative, L and L" may be independently selected from the group consisting of substituted or unsubstituted C₆ to C₃₆ aryl and substituted or unsubstituted C₂ to C₃₅ heteroaryl, and L' may be selected from the group consisting of H, D, C₁ to C₆ alkyl, substituted or unsubstituted C₁ to C₆ alkenyl, substituted or unsubstituted C₆ to C₃₆ aryl, and substituted or unsubstituted C₂ to C₃₅ heteroaryl. In this first alternative, L and L" may be independently selected from the group consisting of substituted or unsubstituted C₆ to C₁₈ aryl and substituted or unsubstituted C₂ to C₁₇ heteroaryl, and L' may be selected from the group consisting of H, D, C₁ to C₄ alkyl, substituted or unsubstituted C₁ to C₄ alkenyl, substituted or unsubstituted C₆ to C₁₈ aryl, and substituted or unsubstituted C₂ to C₁₇ heteroaryl. In this first alternative, L and L" may be independently selected from the group consisting of substituted or unsubstituted phenyl and substituted or unsubstituted C₂ to C₅ heteroaryl, and L' may be selected from the group consisting of H, D, C₁ to C₄ alkyl, substituted or unsubstituted C₁ to C₄ alkenyl, substituted or unsubstituted phenyl, and substituted or unsubstituted C₂ to C₅ heteroaryl.

In this first alternative, L and L" may be independently selected from the group consisting of substituted or unsubstituted C₆ to C₄₈ aryl and substituted or unsubstituted C₂ to C₄₇ heteroaryl, and L' may be selected from the group consisting of H, D, C₁ to C₂₂ alkyl, substituted or unsubstituted C₁ to C₁₂ alkenyl. In this first alternative, L and L" may be independently selected from the group consisting of substituted or unsubstituted C₆ to C₃₆ aryl and substituted or unsubstituted C₂ to C₃₅ heteroaryl, and L' may be selected from the group consisting of H, D, C₁ to C₆ alkyl, substituted or unsubstituted C₁ to C₆ alkenyl. In this first alternative, L and L" may be independently selected from the group consisting of substituted or unsubstituted C₆ to C₁₈ aryl and substituted or unsubstituted C₂ to C₁₇ heteroaryl, and L' may be selected from the group consisting of H, D, C₁ to C₄ alkyl, substituted or unsubstituted C₁ to C₄ alkenyl. In this first alternative, L and L" may be independently selected from the group consisting of substituted or unsubstituted phenyl and substituted or unsubstituted C₂ to C₅ heteroaryl, and L' may be selected from the group consisting of H, D, C₁ to C₄ alkyl, substituted or unsubstituted C₁ to C₄ alkenyl.

In this first alternative, L and L" may be independently selected from the group consisting of substituted or unsubstituted C₆ to C₄₈ aryl, and L' may be selected from the group consisting of H and D. In this first alternative, L and L" may be independently selected from the group consisting of substituted or unsubstituted C₆ to C₃₆ aryl, and L' may be selected from the group consisting of H and D. In this first alternative, L and L" may be independently selected from the group consisting of substituted or unsubstituted C₆ to C₁₈ aryl, and L' may be selected from the group consisting of H and D. In this first alternative, L and L" may be independently selected from the group consisting of substituted or unsubstituted phenyl, and L' may be selected from the group consisting of H and D. In this first alternative, L and L" are independently selected from substituted or unsubstituted C₆ to C₆₀ aryl and L' may be H. In this first alternative, L and L" may be each unsubstituted phenyl, and L' may be H.

In a second alternative, L is selected from the group consisting of substituted or unsubstituted C₆ to C₆₀ aryl and substituted and unsubstituted C₂ to C₆₀ heteroaryl, and L' and L" form together a condensed 6-membered aromatic ring,
wherein the condensed 6-membered aromatic ring
- is unsubstituted,
   or
- is substituted with one or more substituent(s) independently selected from substituted or unsusbtituted C₆ to C₃₆ aryl, provided that the total number of C-atoms of all substituents does not exceed 56, wherein the C₆ to C₅₆ aryl substituents may be further substituted with one or more selected from the group consisting of D, halogen, SiR^{a}R^{b}R^{c} (wherein R^{a}, R^{b} and R^{c} are independently selected from the group consisting of C₁ to C₆ alkyl and phenyl), C₁ to C₆ alkyl, C₁ to C₆ alkoxy, CN and P(=O)R^{d}R^{e} (wherein R^{d} and R^{e} are independently selected from the group consisting of C₁ to C₆ alkyl and phenyl),
   or
- is condensed with a second 6-membered aromatic ring, wherein the condensed 6-membered aromatic ring and the second 6-membered aromatic ring are unsubstituted or at least one of the condensed 6-membered aromatic ring and the second 6-membered aromatic ring is substituted with one or more substituent(s) independently selected from C₆ to C₄₀ aryl provide that the total number of C-atoms of all substituents does not exceed 40, wherein the C₆ to C₄₀ aryl substituents may be further substituted with one or more selected from the group consisting of D, halogen, SiR^{a}R^{b}R^{c} (wherein R^{a}, R^{b} and R^{c} are independently selected from the group consisting of C₁ to C₆ alkyl and phenyl), C₁ to C₆ alkyl, C₁ to C₆ alkoxy, CN and P(=O)R^{d}R^{e} (wherein R^{d} and R^{c} are independently selected from the group consisting of C₁ to C₆ alkyl and phenyl).

For example, in case that L' and L" form together a condensed 6-membered aromatic ring which is unsubstituted, the following structure is formed 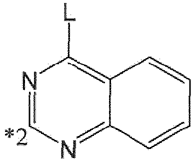 wherein binding to the remaining structure of formula (I) is at *2.

For example, in case that L' and L" form together a condensed 6-membered aromatic ring which is condensed with a second 6-membered aromatic ring, wherein the condensed 6-membered aromatic ring and the second 6-membered aromatic ring are unsubstituted, the following structure may be formed 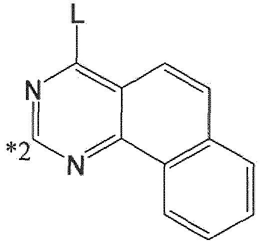 wherein binding to the remaining structure of formula (I) is at *2.

The one or more substituent(s) on L, L' and L" in case that the respective group is substituted are independently selected from the group consisting of D, halogen, SiR^{a}R^{b}R^{c} (wherein R^{a}, R^{b} and R^{c} are independently selected from the group consisting of C₁ to C₆ alkyl and phenyl), C₁ to C₆ alkyl, C₁ to C₆ alkoxy, CN and P(=O)R^{d}R^{e} (wherein R^{d} and R^{e} are independently selected from the group consisting of C₁ to C₆ alkyl and phenyl). This includes substituents on aryl substituents attached to L¹ and L².

In this second alternative, L may be selected from the group consisting of substituted or unsubstituted C₆ to C₄₈ aryl and substituted and unsubstituted C₂ to C₄₇ heteroaryl, and L' and L" may form together a condensed 6-membered aromatic ring,
wherein the condensed 6-membered aromatic ring
- is unsubstituted,
   or
- is substituted with one or more substituent(s) independently selected from substituted or unsubstituted C₆ to C₄₄ aryl, provided that the total number of C-atoms of all substituents does not exceed 44,
   or
- is condensed with a second 6-membered aromatic ring, wherein the condensed 6-membered aromatic ring and the second 6-membered aromatic ring are unsubstituted or at least one of the condensed 6-membered aromatic ring and the second 6-membered aromatic ring is substituted with one or more substituent(s) independently selected from substituted or unsubstituted C₆ to C₂₈ aryl provide that the total number of C-atoms of all substituents does not exceed 28.

In this second alternative, L may be selected from the group consisting of substituted or unsubstituted C₆ to C₁₈ aryl and substituted and unsubstituted C₂ to C₄₇ heteroaryl, and L' and L" may form together a condensed 6-membered aromatic ring,
wherein the condensed 6-membered aromatic ring
- is unsubstituted,
   or
- is substituted with one or more substituent(s) independently selected from substituted or unsubstituted C₆ to C₁₄ aryl, provided that the total number of C-atoms of all substituents does not exceed 14,
   or
- is condensed with a second 6-membered aromatic ring, wherein the condensed 6-membered aromatic ring and the second 6-membered aromatic ring are unsubstituted or at least one of the condensed 6-membered aromatic ring and the second 6-membered aromatic ring is substituted with one or more substituent(s) independently selected from substituted or unsubstituted C₆ to C₁₂ aryl provide that the total number of C-atoms of all substituents does not exceed 12.

In this second alternative, L may be selected from substituted or unsubstituted C₆ to C₆₀ aryl, and L' and L" may form together a condensed 6-membered aromatic ring,
wherein the condensed 6-membered aromatic ring
- is unsubstituted,
   or
- is substituted with one or more substituent(s) independently selected from substituted or unsubstituted C₆ to C₅₆ aryl, provided that the total number of C-atoms of all substituents does not exceed 56.

In this second alternative, L may be selected from the group consisting of substituted or unsubstituted phenyl and substituted and unsubstituted C₂ to C₅ heteroaryl, and L' and L" form together a condensed 6-membered aromatic ring,
wherein the condensed 6-membered aromatic ring
- is unsubstituted,
   or
- is condensed with a second 6-membered aromatic ring, wherein the condensed 6-membered aromatic ring and the second 6-membered aromatic ring are unsubstituted.

In this second alternative, L may be phenyl, and L' and L" form together a condensed 6-membered aromatic ring,
wherein the condensed 6-membered aromatic ring
- is unsubstituted,
   or
- is condensed with a second 6-membered aromatic ring, wherein the condensed 6-membered aromatic ring and the second 6-membered aromatic ring are unsubstituted.

E is independently selected from the group consisting of H, D, halogen, SiR^{a}R^{b}R^{c} (wherein R^{a}, R^{b} and R^{c} are independently selected from the group consisting of C₁ to C₆ alkyl and phenyl), C₁ to C₆ alkyl, C₁ to C₆ alkoxy, CN and P(=O)R^{d}R^{e} (wherein R^{d} and R^{e} are independently selected from the group consisting of C₁ to C₆ alkyl and phenyl).

E may be independently selected from the group consisting of H, D, C, to C₆ alkyl, and C₁ to C₆ alkoxy. E may be independently selected from the group consisting of H, D, and C₁ to C₆ alkyl. E may be independently selected from the group consisting of H and D. Each E may be H.

G is independently selected from the group consisting of H, D, halogen, SiR^{a}R^{b}R^{c} (wherein R^{a}, R^{b} and R^{c} are independently selected from the group consisting of C₁ to C₆ alkyl and phenyl), C₁ to C₆ alkyl, C₁ to C₆ alkoxy, CN and P(=O)R^{d}R^{e} (wherein R^{d} and R^{e} are independently selected from the group consisting of C₁ to C₆ alkyl and phenyl).

G may be independently selected from the group consisting of H, D, C₁ to C₆ alkyl, and C₁ to C₆ alkoxy. G may be independently selected from the group consisting of H, D, and C₁ to C₆ alkyl. G may be independently selected from the group consisting of H and D. Each G may be H.

According to one embodiment, the invention is related to a compound of formula (I) 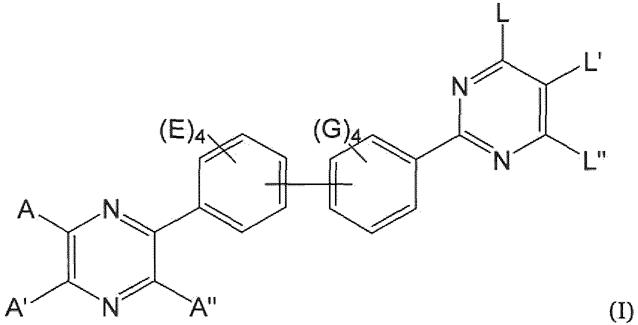 wherein
- A, A' and A" are independently selected from the group consisting of substituted or unsubstituted C₆ to C₆₀ aryl;
- L and L" are independently selected from the group consisting of substituted or unsubstituted C₆ to C₄₈ aryl, and L' is selected from the group consisting of H and D;
   or
- L is selected from the group consisting of substituted or unsubstituted C₆ to C₆₀ aryl, and L' and L" form together a condensed 6-membered aromatic ring,
wherein the condensed 6-membered aromatic ring
- is unsubstituted,
   or
- is condensed with a second 6-membered aromatic ring, wherein the condensed 6-membered aromatic ring and the second 6-membered aromatic ring are substituted or unsubstituted;

- E is independently selected from the group consisting of H, D, C₃ to C₆ alkyl, and C₁ to C₆ alkoxy;
- G is independently selected from the group consisting of H, D, C₁ to C₆ alkyl, and C₁ to C₆ alkoxy.

According to one embodiment, the invention is related to a compound of formula (I) 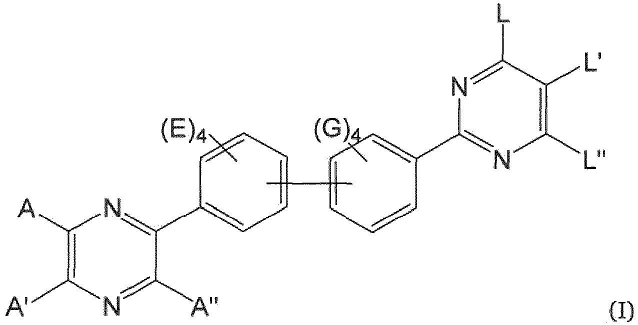 wherein
- A, A' and A" are each unsubstituted phenyl;
- L and L" are each unsubstituted phenyl, and L' is H;
   or
- L is unsubstituted phenyl, and L' and L" form together a condensed 6-membered aromatic ring,
wherein the condensed 6-membered aromatic ring
- is unsubstituted,
   or
- is condensed with a second 6-membered aromatic ring, wherein the condensed 6-membered aromatic ring and the second 6-membered aromatic ring are unsubstituted;

- E are each H;
- G are each H.

The compound of formula (I) may be selected from the following compounds E1 to E7.

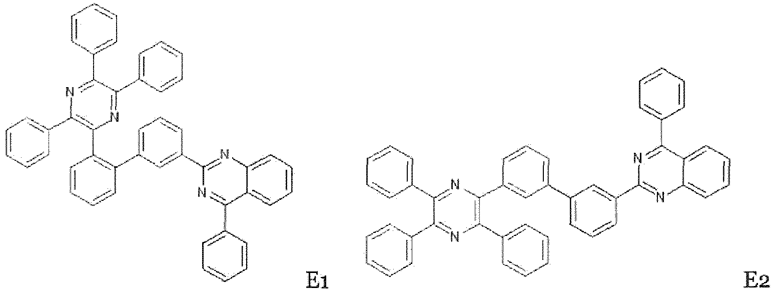

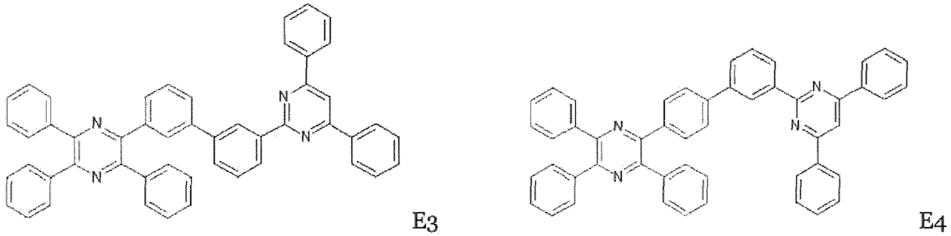

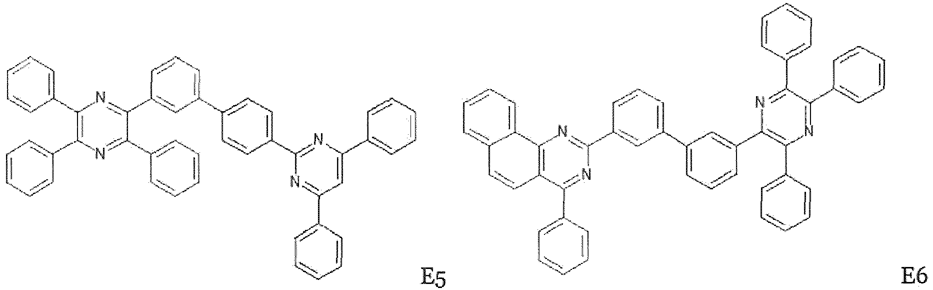

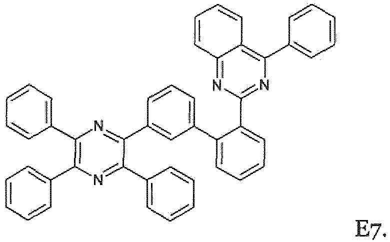

### Semiconducting material

According to one aspect, the invention is related to a semiconducting material comprising the compound of formula (I) in accordance with the invention as defined herein.

The semiconducting material may be an organic semiconducting material. The semiconducting material may be an electron transporting material.

The semiconducting material may comprise the compound of formula (I) in an amount of at least 50 wt.-% with respect to the total weight of the semiconducting material. The semiconducting material may comprise the compound of formula (I) in an amount of at least 60 wt.-% with respect to the total weight of the semiconducting material. The semiconducting material may comprise the compound of formula (I) in an amount of at least 70 wt.-% with respect to the total weight of the semiconducting material. The semiconducting material may comprise the compound of formula (I) in an amount of at least 80 wt.-% with respect to the total weight of the semiconducting material. The semiconducting material may comprise the compound of formula (I) in an amount of at least 90 wt.-% with respect to the total weight of the semiconducting material. The semiconducting material may comprise the compound of formula (I) in an amount of at least 95 wt.-% with respect to the total weight of the semiconducting material. The semiconducting material may comprise the compound of formula (I) in an amount of at least 98 wt.-% with respect to the total weight of the semiconducting material. The semiconducting material may comprise the compound of formula (I) in an amount of at least 99 wt.-% with respect to the total weight of the semiconducting material. The semiconducting material may substantially consist of the compound of formula (I). The semiconducting material may consist of the compound of formula (I).

The semiconducting material may be undoped. Alternatively, the semiconducting material may be doped with an electrical n-type dopant.

Under n-type dopant it is understood a compound which, if embedded into an electron transport matrix material, improves, in comparison with the neat electron transport matrix material under the same physical conditions, the electron properties of the formed semiconducting material, particularly in terms of electron injection and/or electron conductivity.

In the context of the present invention "embedded into an electron transport matrix" means homogenously mixed with the electron transport matrix.

The n-type dopant may be selected from elemental metals, metal salts, metal complexes and organic radicals.

The metal salt can be selected from alkali metal salts and alkaline earth metal salts, in one embodiment from Li, Na, K, R, Cs, Mg, Ca, Sr and Ba salts. The salt may comprise anions selected from halogen anions, complex borate anions, organic phenolate anions. In one embodiment, the anions in the salt may be cation chelating anions, such as 8-hydroxyquinolinolate and/or 2-phosphoryl-phenolate.

In one embodiment, the n-type dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,
- the lithium complex has the formula II, III or IV: 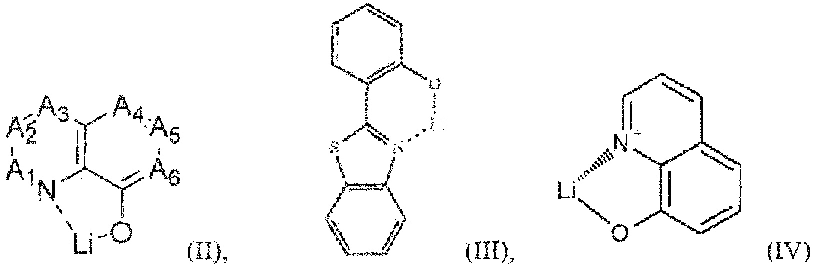 wherein
   A₁ to A₆ are same or independently selected from CH, CR, N, O;
   R is same or independently selected from hydrogen, halogen, alkyl or aryl or heteroaryl with 1 to 20 carbon atoms; and more preferred A₁ to A₆ are CH,

   - the borate based organic ligand is a tetra(1H-pyrazol-1-yl)borate,
   - the phenolate is a 2-(pyridin-2-yl)phenolate, a 2-phosphoryl-phenolate, such as 2-(diphenylphosphoryl)phenolate, an imidazol phenolate, 2-(pyridin-2-yl)phenolate or 2-(1-phenyl-1H-benzo[d]imidazol-2-yl)phenolate,
   - the pyridinolate is a 2-(diphenylphosphoryl)pyridin-3-olate,

- the lithium Schiff base has the structure 100, 101, 102 or 103:

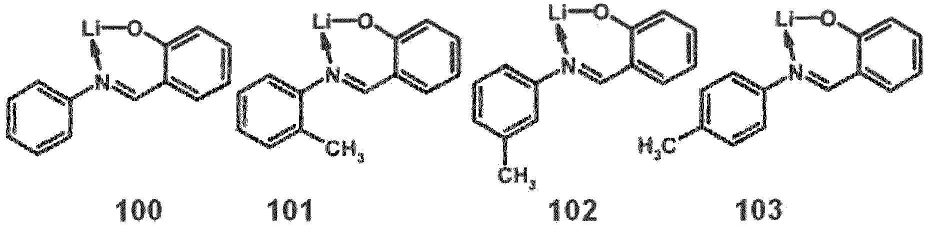

According to one embodiment of the invention, the semiconducting material of the present invention comprises a lithium organic complex, alternatively 8-Hydroxyquinolinolato-lithium (= LiQ).

### Organic electronic device

According to one aspect, the invention is related to an organic electronic device, such as an organic semiconducting device, comprising a semiconducting layer comprising the semiconducting material according to the invention. The semiconducting layer may consist of the semiconducting material according to the invention.

The semiconducting layer may be a hole blocking layer and/or an electron transport layer and/or an electron injection layer. The semiconducting layer may be a hole blocking layer.

The organic electronic device may further comprise an anode, a cathode and a light emission layer, wherein the light emission layer is arranged between the anode and the cathode, and the semiconducting layer is arranged between the light emission layer and the cathode.

The organic electronic device may further comprise an anode, a cathode, a light emission layer and an electron transport layer, wherein the light emission layer and the electron transport layer are arranged between the anode and the cathode, and the semiconducting layer is arranged between the light emission layer and the electron transport layer.

The organic electronic device may further comprise an anode, a cathode, a light emission layer and an electron transport layer, wherein the light emission layer and the electron transport layer are arranged between the anode and the cathode, and the semiconducting layer is arranged between the light emission layer and the electron transport layer, wherein the semiconducting layer is in direct contact with the light emission layer and/or the electron transport layer, preferably is in direct contact with the light emission layer and/or the electron transport layer.

The organic electronic device may further comprise an anode, a cathode, a light emission layer, an electron transport layer and an electron injection layer, wherein the light emission layer, the electron transport layer and the electron injection layer are arranged between the anode and the cathode, and the semiconducting layer is arranged between the light emission layer and the electron transport layer, wherein the semiconducting layer is in direct contact with the light emission layer and/or the electron transport layer, preferably is in direct contact with the light emission layer and/or the electron transport layer.

The organic electronic device may be an organic light emitting diode.

The organic electronic device in accordance with the invention may comprise especially, besides the semiconducting layer comprising or consisting of the semiconducting material according to the invention, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### Anode electrode

Either a first electrode or a second electrode comprised in the inventive organic semiconducting device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO₂), aluminum zinc oxide (AlZO) and zinc oxide (ZnO), may be used to form the anode electrode. The anode electrode may also be formed using metals, typically silver (Ag), gold (Au), or metal alloys.

### Hole injection layer

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

### Hole transport layer

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Photoactive layer (PAL)

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

It may be provided that the photoactive layer does not comprise the compound of Formula (1).

The photoactive layer may be a light-emitting layer or a light-absorbing layer.

### Emission layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (1).

The respective emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA), bis(2-(2-hydroxyphenyl)benzo-thiazolate)zinc (Zn(BTZ)₂), EML3 below, Compound 1 below, and Compound 2 below.

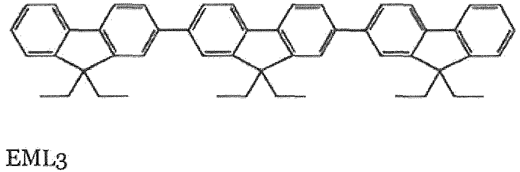

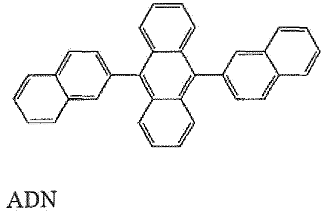

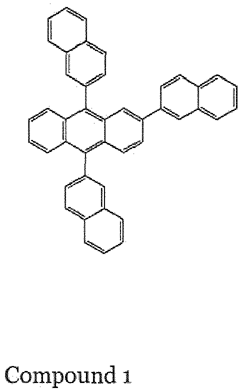

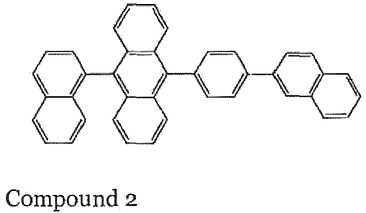

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp₂lr(acac), but are not limited thereto. These compounds are phosphorescent emitters, however, fluorescent red emitter dopants could also be used.

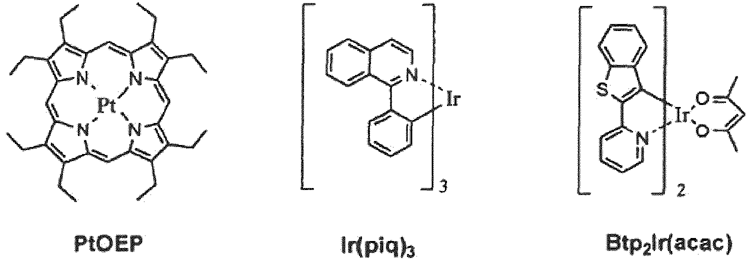

Examples of phosphorescent green emitter dopants are Ir(ppy)₃ (ppy = phenylpyridine), Ir(ppy)₂(acac), Ir(mpyp)₃ are shown below. Compound 3 is an example of a fluorescent green emitter and the structure is shown below.

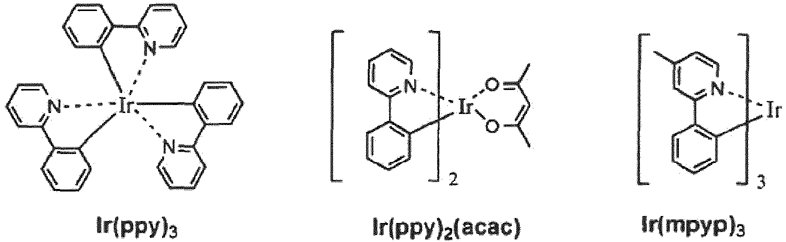

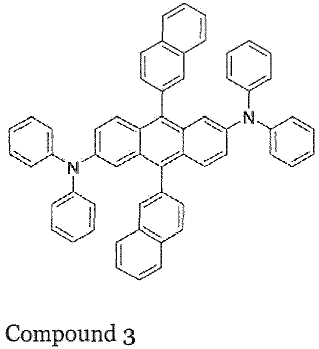

Examples of phosphorescent blue emitter dopants are F2Irpic, (F2ppy)2Ir(tmd) and Ir(dfppz)3, ter-fluorene, the structures are shown below. 4.4'-bis(4-diphenyl amiostyryl)biphenyl (DPAVBi), 2,5,8,11-tetra-tert-butyl perylene (TBPe), and Compound 4 below are examples of fluorescent blue emitter dopants.

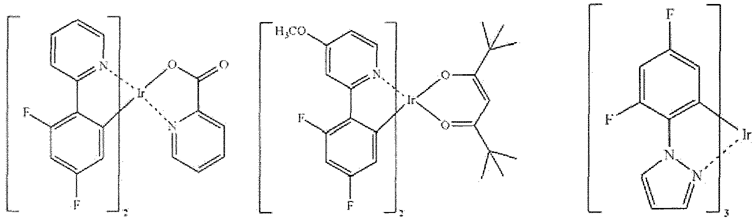

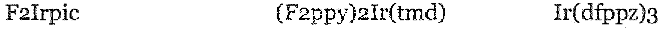

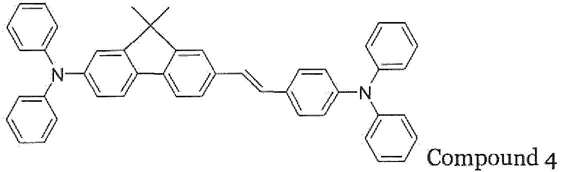

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or α-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

The hole blocking layer may be made of a semiconducting material according to the invention.

### Electron transport layer (ETL)

The semiconducting device according to the present invention may comprise an electron transport layer (ETL).

According to various embodiments the OLED may comprise an electron transport layer or an electron transport layer stack comprising at least a first electron transport layer and at least a second electron transport layer.

By suitably adjusting energy levels of particular layers of the ETL, the injection and transport of the electrons may be controlled, and the holes may be efficiently blocked. Thus, the OLED may have long lifetime.

The electron transport layer may comprise ETM materials comprising one or more electron transport compound(s) known in the art.

According to an embodiment, the electron transport layer comprises an electron transport compound, wherein the electron transport compound comprises 8 to 13 aromatic or heteroaromatic rings, optionally 8 to 11 aromatic or heteroaromatic rings, optionally 9 to 11 aromatic or heteroaromatic rings, and optionally 9 aromatic or heteroaromatic rings, wherein one or more of the aromatic or heteroaromatic rings may be substituted with C₁ to C₄ alkyl. In this regard, an aromatic, respectively heteroaromatic ring is a single aromatic ring, for example a 6-membered aromatic ring such as phenyl, a 6-membered heteroaromatic ring such as pyridyl, a 5-membered heteroaromatic ring such as pyrrolyl etc. In a system of condensed (hetero)romatic rings, each ring is considered as a single ring in this regard. For example, naphthalene comprises two aromatic rings.

The electron transport compound may comprise at least one heteroaromatic ring, optionally 1 to 5 heteroaromatic rings, optionally 1 to 4 heteroaromatic rings, optionally 1 to 3 heteroaromatic rings, and optionally 1 or 2 heteroaromatic rings.

The aromatic or heteroaromatic rings of the electron transport compound may be 6-membered rings.

The heteroaromatic rings of the electron transport compound may be a N-containing heteroaromatic ring, optionally allof the heteroaromatic rings are N-containing heteroaromatic rings, optionally all of the heteroaromatic rings heteroaromatic rings contain N as the only type of heteroatom.

The electron transport compound may comprise at least one six-member heteroaromatic ring containing one to three N-atoms in each heteroaromatic ring, optionally one to three 6-membered heteroaromatic rings containing one to three N-atoms in each heteroaromatic ring, respectively.

The at least one 6-membered heteroaromatic ring comprised in the electron transport compound may be an azine. The at least one 6-membered heteroaromatic ring comprised in the electron transport compound may be triazine, diazine, pyrazine, pyrimidine, pyridine, quinazoline or bonzoquinazoline, preferably triazine.

If the electron transport compound comprises two or more heteroaromatic rings, the heteroaromatic rings may be separated from each other by at least one aromatic ring which is free of a heteroatom.

In an embodiment, the heteroatoms in the heteroaromatic rings of the electron transport compound are bound into the molecular structure of the electron transport compound by at least one double bond.

Further, the electron transport layer may comprise one or more additives. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another emdodiment, the n-type dopant can be one selected from a group consisting of Cs, K, Rb, Mg, Na, Ca, Sr, Eu and Yb. In an embodiment the alkali metal compound may be 8-Hydroxyquinolinolato-lithium (LiQ), Lithium tetra(1H-pyrazol-1-yl)borate or Lithium 2-(diphenylphosphoryl)phenolate. Suitable compounds for the ETM (which may be used in addition to the inventive compound represented by the general Formula (1) as defined above) are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.

The electron transport layer may comprise the compound of formula (I) according to the invention.

### Electron injection layer (EIL)

The optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li₂O BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

The electron injection layer may comprise the compound of formula (I) according to the invention.

### Cathode electrode

The cathode electrode is formed on the EIL if present. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO. The cathode may comprise more that 50 volume % of metal selected from Ag and Au.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy.

The cathode may be a semi-transparent metal cathode having a thickness less than 20 nm, preferably less than 15 nm, even more preferably less than 12 nm.

It is to be understood that the cathode electrode is not part of an electron injection layer or the electron transport layer.

### Charge generation layer/hole generation layer

The charge generation layer (CGL) is composed of a double layer.

The charge generation layer is a pn junction joining an n-type charge generation layer (electron generation layer) and a p-type charge generation layer (hole generation layer). The n-side of the pn junction generates electrons and injects them into the layer which is adjacent in the direction to the anode. Analogously, the p-side of the p-n junction generates holes and injects them into the layer which is adjacent in the direction to the cathode.

Charge generation layers are used in tandem devices, for example, in tandem OLEDs comprising, between two electrodes, two or more emission layers. In a tandem OLED comprising two emission layers, the n-type charge generation layer provides electrons for the first light emission layer arranged near the anode, while the p-type charge generation layer provides holes to the second light emission layer arranged between the first emission layer and the cathode.

The hole generation layer can be composed of an organic matrix material doped with p-type dopant. Suitable matrix materials for the hole generation layer may be materials conventionally used as hole injection and/or hole transport matrix materials. Also, p-type dopant used for the hole generation layer can employ conventional materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, FeCl3, FeF3, and SbCl5. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB).

The n-type charge generation layer can be layer of a neat n-dopant, for example of an electropositive metal, or can consist of an organic matrix material doped with the n-dopant. In one embodiment, the n-type dopant can be alkali metal, alkali metal compound, alkaline earth metal, or alkaline earth metal compound. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline)aluminum, benzazole derivatives, and silole derivatives.

In one embodiment, the p-type charge generation layer may include compounds of the following Chemical Formula X. 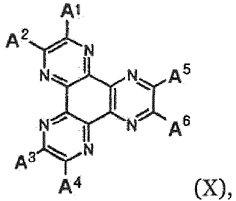 wherein each of A1 to A6 may be hydrogen, a halogen atom, nitrile (-CN), nitro (-NO2), sulfonyl (-SO2R), sulfoxide (-SOR), sulfonamide (-SO2NR), sulfonate (-SO3R), trifluoromethyl (-CF3), ester (-COOR), amide (-CONHR or - CONRR'), substituted or unsubstituted straight-chain or branched-chain C1-C12 alkoxy, substituted or unsubstituted straight-chain or branched-chain C1-C12 alkyl, substituted or unsubstituted straight-chain or branched chain C2-C12 alkenyl, a substituted or unsubstituted aromatic or non-aromatic heteroring, substituted or unsubstituted aryl, substituted or unsubstituted mono- or di-arylamine, substituted or unsubstituted aralkylamine, or the like. Herein, each of the above R and R' may be substituted or unsubstituted C1-C60 alkyl, substituted or unsubstituted aryl, or a substituted or unsubstituted 5- to 7-membered heteroring, or the like.

An example of such p-type charge generation layer may be a layer comprising CNHAT

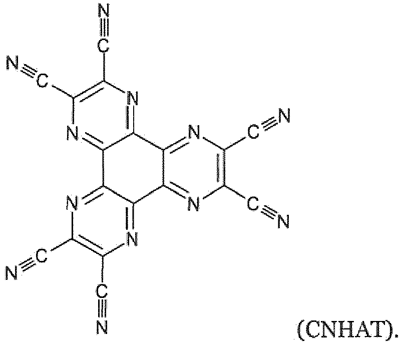

The hole generating layer may be arranged on top of the n-type charge generation layer.

With regard to the method for producing the organic electronic device, the alternative embodiments outlined above may be applied mutatis mutandis. For example, the charge generation layer may be produced with an interlayer provided between the n-type sub-layer, and the p-type sub-layer.

According to one aspect, the OLED according to the present invention can comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer (n-type sub-layer), the n-type charge generation layer is adjacent arranged to a hole generating layer (p-type sub-layer), an interlayer may be provided between the n-type sub-layer, and the p-type sub-layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

### Display device

According to a further aspect, the invention is related to a display device comprising the organic electronic device according to the invention, wherein the organic electronic device is an organic light emitting device.

### Process for preparing the organic electronic device

According to a further aspect, the invention is related to a process for preparing the organic electronic device according to the present invention, wherein the process comprises a step of depositing the compound of formula (I) according to the present invention on a solid support.

The method for depositing may comprise:
- deposition via vacuum thermal evaporation;
- deposition via solution processing, preferably the processing is selected from spin-coating, printing, casting; and/or
- slot-die coating.

### Details and definitions of the invention

An organic compound as referred to herein is generally any chemical compound that contains carbon (except some compounds generally referred to as being inorganic, such as carbonates, cyanides, carbon dioxide, diamond etc.). The term organic compound used herein also encompasses compounds such as organometallic compounds, for example metallocenes etc.

If not mentioned else explicitly, all compounds, groups, moieties, substituents etc. shown herein, especially by structural formulas, by systematic names etc. encompass the respective partially and fully deuteriated derivatives thereof.

The term "zero-valent" as used herein refers to a metal in the oxidation state 0, i.e. particular to metals from which no electron has been removed. The zero-valent metal may be present in the form of zero-valent atoms, neat metal, alloys etc.

The term "trivalent" as used herein refers to a nitrogen atom with a single bond and a double bond and containing a lone pair of electrons.

The term "hydrocarbyl group" as used herein shall be understood to encompass any organic group comprising carbon atoms, in particular organic groups, such as alkyl, aryl, heteroaryl, heteroalkyl, in particular such groups which are substituents usual in organic electronics.

The term "conjugated system" as used herein refer to a system of alternating π- and σ-bonds or a molecule having alternating single and multiple bonds i.e. double bond or a system having one or more two-atom structural units having the π-bond between its atoms can be replaced by an atom bearing at least one lone electron pair, typically by a divalent O or S atom.

The term "alkyl" as used herein shall encompass linear as well as branched and cyclic alkyl. For example, C3-alkyl may be selected from n-propyl and iso-propyl. Likewise, C4-alkyl encompasses n-butyl, see-butyl and t-butyl. Likewise, C6-alkyl encompasses n-hexyl and cyclohexyl.

The subscribed number n in Cn relates to the total number of carbon atoms in the respective alkyl, arylene, heteroarylene or aryl group.

The term "aryl" or "arylene" as used herein shall encompass phenyl (C₆-aryl), fused aromatics, such as naphthalene, anthracene, phenanthrene, tetracene etc.. Further encompassed are biphenyl and oligo- or polyphenyls, such as terphenyl, phenyl-substituted biphenyl, phenyl-substituted terphenyl (such as tetraphenyl benzole groups) etc.. "Arylene" respectively "heteroarylene", refers to groups to which two further moieties are attached. In the present specification, the term "aryl group" or "arylene group" may refer to a group comprising at least one hydrocarbon aromatic moiety, and all the elements of the hydrocarbon aromatic moiety may have p-orbitals which form conjugation, for example a phenyl group, a napthyl group, an anthracenyl group, a phenanthrenyl group, a pyrinyl group, a fluorenyl group and the like. Further encompoassed are spiro compounds in which two aromatic moieties are connected with each other via a spiro-atom, such as 9,9'-spirobi[9H-fluorene]yl. The aryl or arylene group may include a monocyclic or fused ring polycyclic (i.e., links sharing adjacent pairs of carbon atoms) functional group.

The term "heteroaryl" as used herein refers to aryl groups in which at least one carbon atom is substituted with a heteroatom. The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms. Preferably, a heteroarylene ring may comprise at least 1 to 3 heteroatoms individually selected from N, S and/or O. Just as in case of "aryl"/"arylene", the term "heteroaryl" comprises, for example, spiro compounds in which two aromatic moieties are connected with each other, such as spiro[fluorene-9,9'-xanthene]. Further exemplary heteroaryl groups are diazine, triazine, dibenzofurane, dibenzothiofurane, acridine, benzoacridine, dibenzoacridine etc.

The subscripted number n in Cₙ-heteroaryl merely refers to the number of carbon atoms excluding the number of heteroatoms. In this context, it is clear that a C₃ heteroarylene group is an aromatic compound comprising three carbon atoms, such as pyrazol, imidazole, oxazole, thiazole and the like.

The term "halogenated" refers to an organic compound in which one hydrogen atom thereof is replaced by a halogen atom. The term "perhalogenated" refers to an organic compound in which all of the hydrogen atoms thereof are replaced by halogen atoms. The meaning of the terms "fluorinated" and "perfluorinated" should be understood analogously.

The term "alkenyl" as used herein refers to a group -CR¹ = CR²R³ comprising a carbon-carbon double bond.

The term "perhalogenated" as used herein refers to a hydrocarbyl group wherein all of the hydrogen atoms of the hydrocarbyl group are replaced by halogen (F, Cl, Br, I) atoms.

The term "alkoxy" as used herein refers to a structural fragment of the Formula -OR with R being hydrocarbyl, preferably alkyl or cycloalkyl.

The term "thioalkyl" as used herein refers to a structural fragment of the Formula -SR with R being hydrocarbyl, preferably alkyl or cycloalkyl.

In the present specification, the term single bond refers to a direct bond.

In terms of the invention, a group is "substituted with" another group if one of the hydrogen atoms comprised in this group is replaced by another group, wherein the other group is the substituent.

In accordance with the present disclosure, in a formula showing the following binding situation, 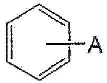 the group A may be bound to any suitable binding position. In a situation were it is shown that the bond of A crosses more than one ring 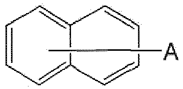 the group A may be bound to any suitable binding position of each ring crossed with the bond.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

With respect to the inventive organic light emitting device, the compounds mentioned in the experimental part may be most preferred.

The organic electroluminescent device (OLED) may be a bottom- or top-emission device.

Another aspect is directed to a device comprising at least one organic electroluminescent device (OLED). A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural formula.

The energy levels of the highest occupied molecular orbital, also named HOMO, and of the lowest unoccupied molecular orbital, also named LUMO, are measured in electron volt (eV).

The terms "OLED" and "organic light-emitting diode" are simultaneously used and have the same meaning. The term "organic electroluminescent device" as used herein may comprise both organic light emitting diodes as well as organic light emitting transistors (OLETs).

As used herein, "weight percent", ,wt.-%", wt%, "percent by weight", "% by weight", and variations thereof refer to a composition, component, substance or agent as the weight of that component, substance or agent of the respective electron transport layer divided by the total weight of the respective electron transport layer thereof and multiplied by 100. It is understood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of", "does not contain", "does not comprise" does not exclude impurities. Impurities have no technical effect with respect to the object achieved by the present invention. The term "free of" a compound means that such compound/material is not deliberately added to the layer during processing.

Preferably, the semiconducting layer comprising the compound of Formula (I) is essentially non-emissive or non-emitting.

The operating voltage, also named U, is measured in Volt (V) at 10 milliAmpere per square centimeter (mA/cm²).

The candela per Ampere efficiency, also named cd/A efficiency is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color. Efficiency values are compared at the same CIE-y.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device" and "organic light-emitting diode" are simultaneously used and have the same meaning.

The term "life-span" and "lifetime" are simultaneously used and have the same meaning.

The anode and cathode may be described as anode electrode / cathode electrode or anode electrode / cathode electrode or anode electrode layer / cathode electrode layer.

Room temperature, also named ambient temperature, is 23° C.

Hereinafter, the embodiments are illustrated in more detail with reference to examples. However, the present disclosure is not limited to the following examples. Reference will now be made in detail to the exemplary aspects.

### DESCRIPTION OF THE DRAWINGS

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
FIG. 1 is a schematic sectional view of an organic semiconducting device, according to an exemplary embodiment of the present invention;
FIG. 2 is a schematic sectional view of an organic light-emitting diode (OLED), according to an exemplary embodiment of the present invention;
FIG. 3 is a schematic sectional view of an OLED, according to an exemplary embodiment of the present invention.
FIG. 4 is a schematic sectional view of an OLED comprising a charge generation layer and two emission layers, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

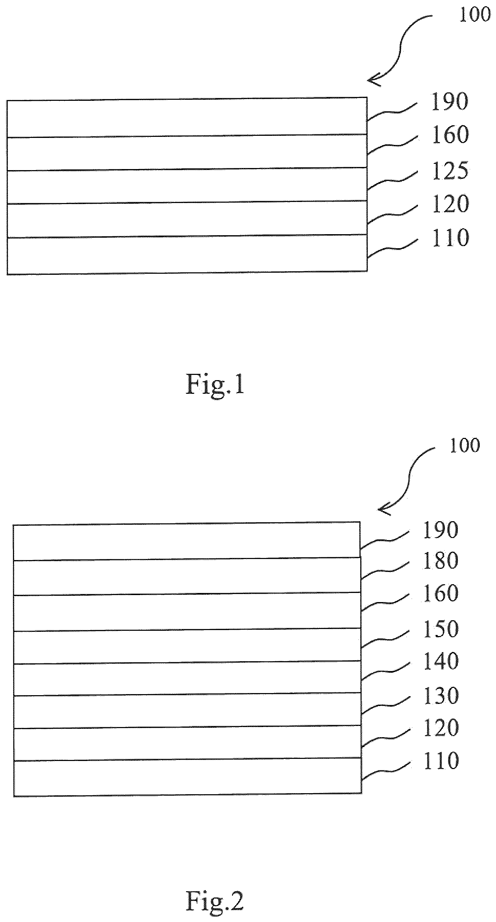

FIG. 1 is a schematic sectional view of an organic semiconducting device 100, according to an exemplary embodiment of the present invention. The organic semiconducting device 100 includes a substrate 110, an anode 120, a light emission layer (EML) 125, a semiconducting layer comprising or consisting of the semiconducting material according to the invention 160. The semiconducting layer comprising or consisting of the semiconducting material according to the invention 160 is formed on the EML 125. Onto the organic semiconductor layer 160, a cathode 190 is disposed.

FIG. 2 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an electron transport layer (ETL) 160. In this embodiment, the electron transport layer is the semiconducting layer according to the invention. The electron transport layer (ETL) 160 is formed on the EML 150. Onto the electron transport layer (ETL) 160, an electron injection layer (EIL) 180 is disposed. The cathode 190 is disposed directly onto the electron injection layer (EIL) 180.

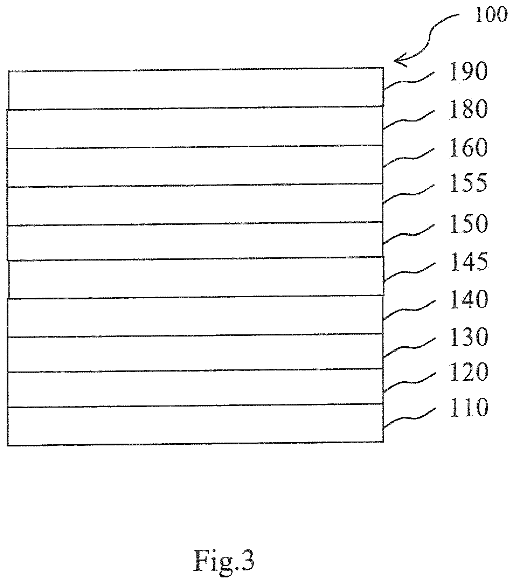

Fig. 3 is a schematic sectional view of an OLED 100, according to another exemplary embodiment of the present invention. Fig. 3 differs from Fig. 2 in that the OLED 100 of Fig. 3 comprises an electron blocking layer (EBL) 145 and a hole blocking layer (HBL) 155.

Referring to Fig. 3, the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode electrode 190.

In this embodiment of Fig. 3, the HBL 155 is the semiconducting layer comprising the compound of formula (I).

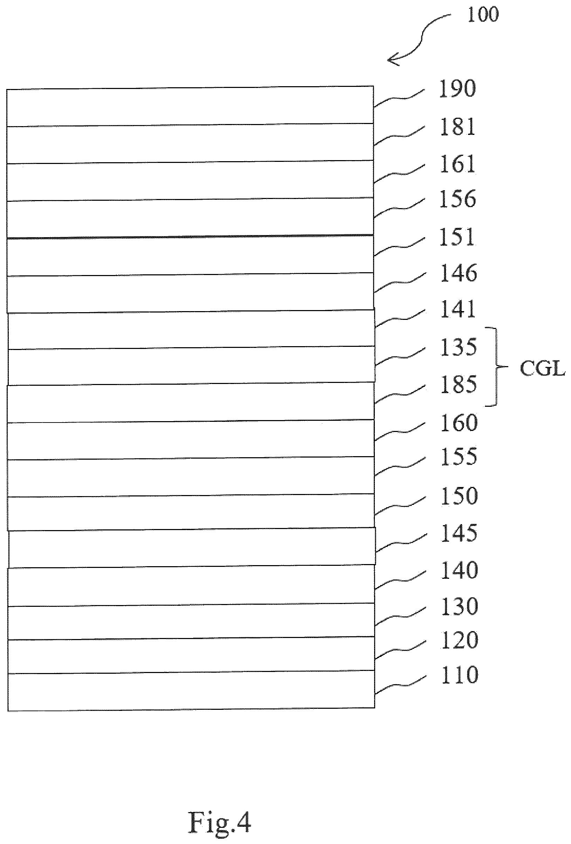

Fig. 4 is a schematic sectional view of an OLED 100, according to another exemplary embodiment of the present invention. Fig. 4 differs from Fig. 3 in that the OLED 100 of Fig. 4 further comprises a charge generation layer (CGL) and a second emission layer (151).

Referring to Fig. 4, the OLED 100 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (EBL) 156, a second electron transport layer (ETL) 161, a second electron injection layer (EIL) 181 and a cathode 190.

In this embodiment of Fig. 4, the HBL 155 is the semiconducting layer comprising the compound of formula (I).

While not shown in Fig. 1, Fig. 2, Fig. 3 and Fig. 4, a sealing layer may further be formed on the cathode electrodes 190, in order to seal the OLEDs 100. In addition, various other modifications may be applied thereto.

Hereinafter, the embodiments are illustrated in more detail with reference to examples. However, the present disclosure is not limited to the following examples.

### Synthesis procedures

### 4-phenyl-2-(2'-(3,5,6-triphenylpyrazin-2-yl)-[1,1'-biphenyl]-3-yl)quinazoline (E1)

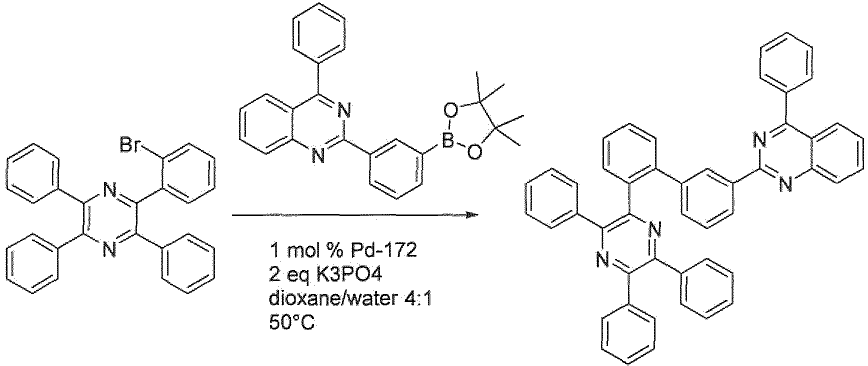

A 3-neck round-bottom flask was flushed with nitrogen and charged with 2-(2-bromophenyl)-3,5,6-triphenylpyrazine (CAS 2368824-18-2, 1 eq, 11.9 g), 4-phenyl-2-(3-(4,4,5,5-tetramethyl-1,3,2-dioxaborolan-2-yl)phenyl)quinazoline (CAS 1852465-26-9, 0.95 eq, 12.8 g), chloro(crotyl)(2-dicyclohexylphosphino-2',6'-dimethoxybiphenyl)palladium(II) (CAS 1798781-99-3, 0.01 eq, 0.2 g), potassium phosphate (CAS 7778-53-2, 2 eq, 12.4 g). A deaerated mixture of 120 mL dioxane and 30 mL water was added. The reaction mixture was stirred at 50 °C under nitrogen atmosphere overnight. Then the reaction mixture was cooled down, the solvent was evaporated. The raw product was extracted in 250 mL chloroform, washed with water. Combined organic phases were dried and filtered through a silica pad. The solvent was partially evaporated under reduced pressure and hexane was added. White solid precipitate was filtered. Final purification was done by sublimation and product obtained as a white crystalline solid. Yield: 11.5 g (67 %); ESI-MS: 665

### 4-phenyl-2-(3'-(3,5,6-triphenylpyrazin-2-yl)-[1,1'-biphenyl]-3-yl)quinazoline (E2)

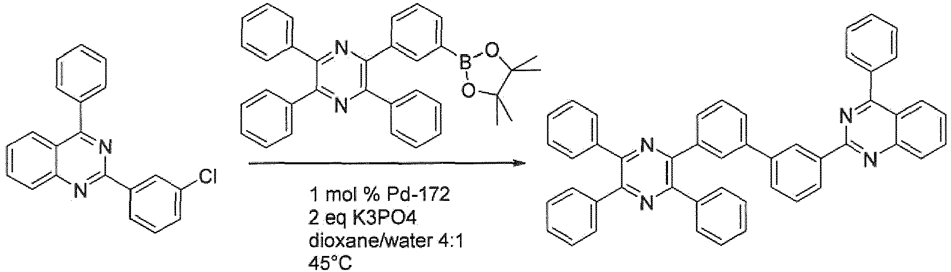

A 3-neck round-bottom flask was flushed with nitrogen and charged with 2-(3-chlorophenyl)-4-phenylquinazoline (CAS 540466-41-9, 1 eq, 25.0 g), 2,3,5-triphenyl-6-(3-(4,4,5,5-tetramethyl-1,3,2-dioxaborolan-2-yl)phenyl)pyrazine (CAS 2396743-64-7, 1.1 eq, 44.3 g), chloro(crotyl)(2-dicyclohexylphosphino-2',6'-dimethoxybiphenyl)palladium(II) (CAS 1798781-99-3, 0.01 eq, 0.5 g), potassium phosphate (CAS 7778-53-2, 2 eq, 33.5 g). A deaerated mixture of 440 mL dioxane and 80 mL water was added. The reaction mixture was stirred at 45 °C under nitrogen atmosphere overnight. After cooling down to room temperature, a brown suspension formed. The raw solid material was filtered, washed with dioxane and water. Product was dissolved at elevated temperature in 1.1 L toluene and filtered hot through a silica pad. First fraction of the filtrate was discarded and the pad was additionally eluted with excess dichloromethane. Then the solvent was evaporated and the raw product was refluxed in 300 mL methyl-tert-butyl ether and filtered. Final purification was done by sublimation, the product obtained as a white powder. Yield: 26.4 g (52 %); ESI-MS: 665

### 4,6-diphenyl-2-(3'-(3,5,6-triphenylpyrazin-2-yl)-[1,1'-biphenyl]-3-yl)pyrimidine (E3)

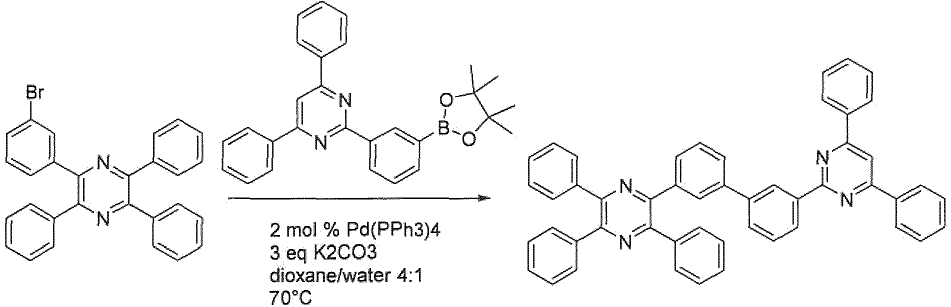

A 3-neck round-bottom flask was flushed with nitrogen and charged with 2-(3-bromophenyl)-3,5,6-triphenylpyrazine (CAS 2368824-17-1) 1 eq (18,9 g), 4,6-diphenyl-2-(3-(4,4,5,5-tetramethyl-1,3,2-dioxaborolan-2-yl)phenyl)pyrimidine (CAS 1381862-91-4, 1 eq, 20.1 g), tetrakis(triphenylphosphine)palladium(0) (CAS 14221-01-3, 0.2 eq, 1.0 g), potassium carbonate (CAS 584-08-7, 3 eq, 18.0 g). A deaerated mixture of 260 mL dioxane, 65 mL water was added. The reaction mixture was stirred at 70 °C under nitrogen atmosphere overnight. After cooling down to room temperature, a dark-grey suspension formed. The raw solid material was filtered, washed with dioxane, water and methanol. Then the product was extracted in Soxhlet apparatus with toluene. After cooling the extract down, a white precipitate formed, which was subsequently filtered and washed with hexane. Then the product was washed in 300 mL hot toluene. Final purification was done by sublimation, the product obtained as a white powder. Yield: 23.9 g (79 %); ESI- MS: 691

### 4,6-diphenyl-2-(4'-(3,5,6-triphenylpyrazin-2-yl)-[1,1'-biphenyl]-3-yl)pyrimidine (E4)

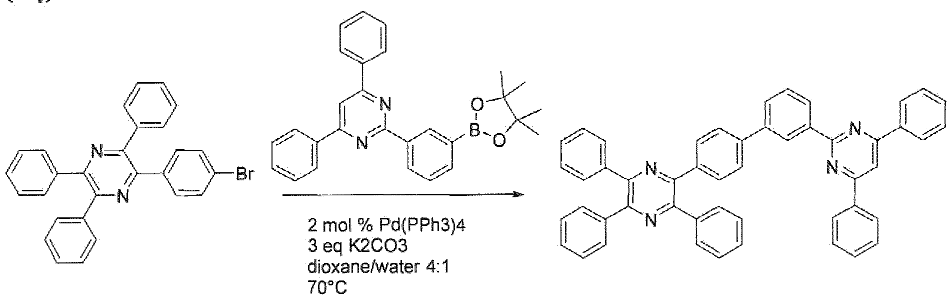

A 3-neck round-bottom flask was flushed with nitrogen and charged with 2-(4-bromophenyl)-3,5,6-triphenylpyrazine (CAS 943442-81-7) 1 eq (18,9 g), 4,6-diphenyl-2-(3-(4,4,5,5-tetramethyl-1,3,2-dioxaborolan-2-yl)phenyl)pyrimidine (CAS 1381862-91-4, 1 eq, 20.1 g), tetrakis(triphenylphosphine)palladium(o) (CAS 14221-01-3, 0.02 eq, 1.0 g), potassium carbonate (CAS 584-08-7, 3 eq, 18.0 g). A deaerated mixture of 260 mL dioxane and 65 mL water was added. The reaction mixture was stirred at 70 °C under a nitrogen atmosphere overnight. After cooling down to room temperature, a gray suspension formed. The raw solid material was filtered, washed with dioxane and water. Then the product was extracted in Soxhlet apparatus with toluene. After cooling the extract down, a white precipitate formed. The solid was filtered and washed with hexane. Final purification was done by sublimation, the product obtained as a white powder. Yield: 23.9 g (79 %); ESI- MS: 691

### 4,6-diphenyl-2-(3'-(3,5,6-triphenylpyrazin-2-yl)-[1,1'-biphenyl]-4-yl)pyrimidine (E5)

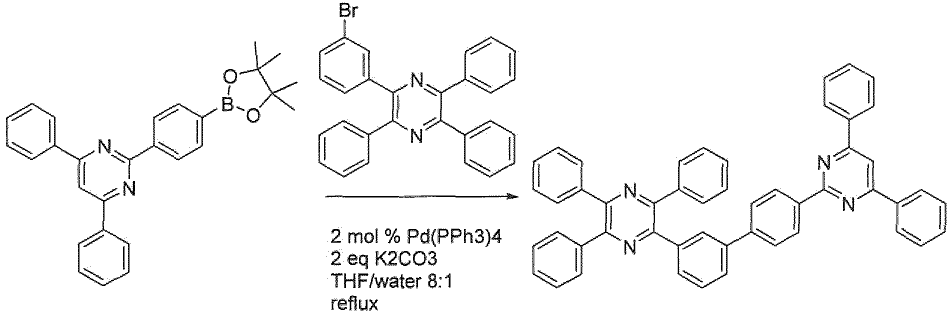

A 3-neck round-bottom flask was flushed with nitrogen and charged with 4,6-diphenyl-2-(4-(4,4,5,5-tetramethyl-1,3,2-dioxaborolan-2-yl)phenyl)pyrimidine (CAS 1613163-88-4, 1 eq, 25.0 g), 2-(3-bromophenyl)-3,5,6-triphenylpyrazine (CAS 2368824-17-1, 1 eq, 23.4 g), tetrakis(triphenylphosphine)palladium(o) (CAS 14221-01-3, 0.02 eq, 1.3 g), potassium carbonate (CAS 584-08-7, 2 eq, 14.9 g). A deaerated mixture of 480 mL THF and 60 mL water was added. The reaction mixture was stirred at 75 °C under nitrogen atmosphere overnight. After cooling down to room temperature, a yellow suspension formed. The raw solid material was filtered, washed with THF and water. Then the product was dissolved in 800 mL chloroform and filtered through a silica pad. Resulting solution was partially evaporated and the mixture of 5% methanol in hexane was added. White precipitate formed and the solid was filtered. Final purification was done by sublimation, the product obtained as a white powder. Yield: 18.9 g (51 %), ESI-MS: 691

### 4-phenyl-2-(3'-(3,5,6-triphenylpyrazin-2-yl)-[1,1'-biphenyl]-3-yl)benzo[h]quinazoline (E6)

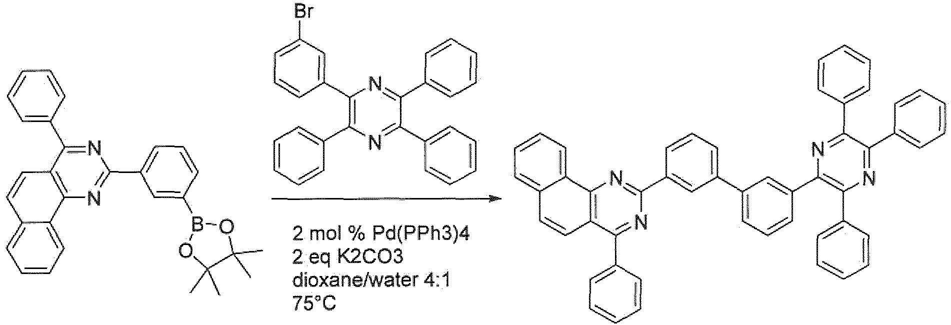

A 3-neck round-bottom flask was flushed with nitrogen and charged with 4-phenyl-2-(3-(4,4,5,5-tetramethyl-1,3,2-dioxaborolan-2-yl)phenyl)benzo[h]quinazoline (CAS 2639481-16-4, 1 eq, 20.0 g), 2-(3-bromophenyl)-3,5,6-triphenylpyrazine (CAS 2368824-17-1, 1 eq, 20.2 g), tetrakis(triphenylphosphine)palladium(o) (CAS 14221-01-3, 0.02 eq, 1.0 g), potassium carbonate (CAS 584-08-7, 3 eq, 12.1 g). A deaerated mixture of 200 mL dioxane and 50 mL water was added. The reaction mixture was stirred at 75 °C under nitrogen atmosphere overnight. After cooling down to room temperature, a dark-gray suspension formed. The raw solid material was filtered, washed with dioxane, water and methanol. Then the product was extracted in Soxhlet apparatus with chlorobenzene. After cooling the extract down white precipitate was formed, filtered and washed with hexane. The solid was washed in 400 mL hot THF and filtered. The product was recrystallized from chlorobenzene. Final purification was done by sublimation, the product obtained as a white powder. Yield: 18.4 g (59 %). ESI-MS: 715

### 4-phenyl-2-(3'-(3,5,6-triphenylpyrazin-2-yl)-[1,1'-biphenyl]-2-yl)quinazoline (E7)

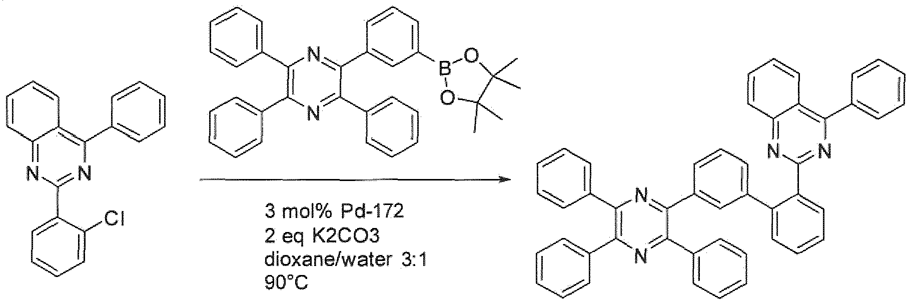

A 3-neck round-bottom flask was flushed with nitrogen and charged with 2-(2-chlorophenyl)-4-phenylquinazoline (CAS 1283751-33-6, 1 eq, 12.8 g), 2,3,5-triphenyl-6-(3-(4,4,5,5-tetramethyl-1,3,2-dioxaborolan-2-yl)phenyl)pyrazine (CAS 2396743-64-7, 1.05 eq, 21.7 g), chloro(crotyl)(2-dicyclohexylphosphino-2',6'-dimethoxybiphenyl)palladium(II) (CAS 1798781-99-3, 0.03 eq, 7.3 g), potassium phosphate (CAS 7778-53-2, 2 eq, 17.2 g). A deaerated mixture of 300 mL dioxane, 100 mL water was added. The reaction mixture was stirred at 90 °C under nitrogen atmosphere overnight. After cooling down to room temperature, gray suspension formed. The raw solid material was filtered, washed with dioxane, water and methanol. Then the product was dissolved in dichloromethane and filtered through a silica pad. Resulting solution was partially evaporated and methanol was added. White precipitate was formed and filtered. Final purification was done by sublimation, the product obtained as a white powder. Yield: 15.4 g (57 %). ESI-MS: 665

### Supporting materials for device experiments

F1 is 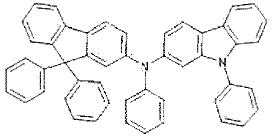 CAS 1607480-22-7
F2 is 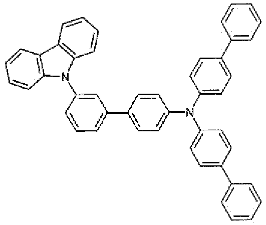 CAS 1464822-27-2
F3 is 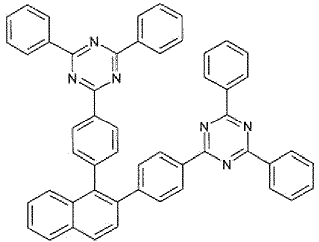 CAS 2244287-14-5
**F4** is 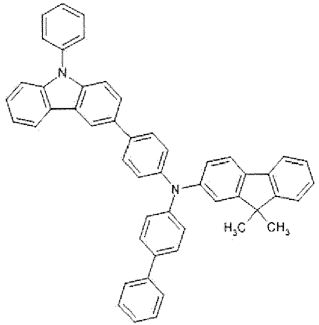 CAS 1242056-42-3 .
**PD2** is 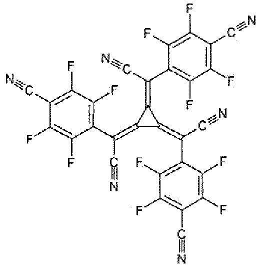 CAS 1224447-88-4
LiQ is lithium 8-hydroxyquinolinolate, CAS 850918-68-2
BH1 is 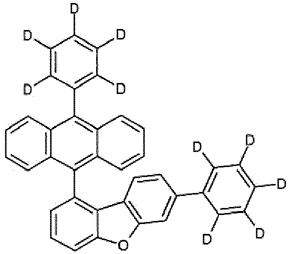 CAS 2457172-82-4
BD1 is 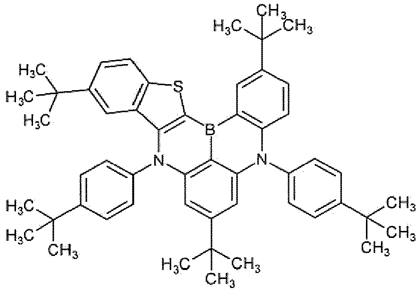 CAS 2482607-57-6
ITO is indium tin oxide.

### Comparative compounds

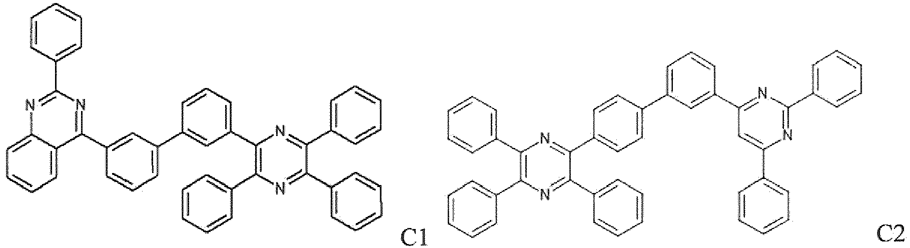

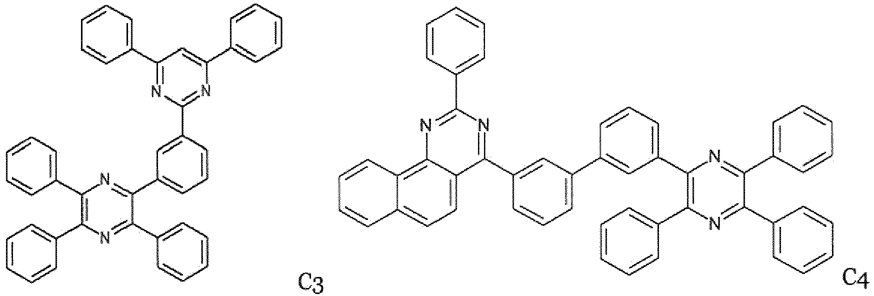

### OLED Devices

### Blue fluorescent top emission OLED

Table 1a schematically describes the model device used for the described OLED tests.

**Table 1:**

| Layer | Material | concentration [vol%] | Layer thickness [nm] |
|---|---|---|---|
| anode | ITO/Ag/ITO | 100/100/100 | 10/120/10 |
| HIL | F1:PD-2 | 97.5:2.5 | 10 |
| HTL | F1 | 100 | 128 |
| EBL | F2 | 100 | 5 |
| EML | BH1:BD1 | 99:1 | 20 |
| HBL | Tested or comparative | 100 | 5 |
| ETL | F3:LiQ | 50:50 | 31 |
| EIL | Yb | 100 | 2 |
| cathode | Ag:Mg | 90:10 | 13 |
| Cap layer | F4 | 100 | 75 |

The results are given in Tables 2 to 5

**Table 2**

| **HBL** | **CIEy** | **Voltage, 10mA/cm² [V]** | **Ceff/CIEy,** 1**0mA/cm² [cd/A]** | **LT97, 30mA/cm² [h]** |
|---|---|---|---|---|
| C1 | 0.050 | 3.60 | 197 | **11** |
| E1 | 0.050 | 3.45 | 205 | 50 |
| E2 | 0.051 | 3.55 | 204 | 102 |

In comparison with state-of-art compound C1, the inventive compounds enable longer lifetime and higher current efficiency.

**Table 3**

| **HBL** | **CIEy** | **Voltage, 10mA/cm² [V]** | **Ceff/CIEy**, **10mA/cm² [cd/A]** | **LT97, 30mA/cm² [h]** |
|---|---|---|---|---|
| C2 | 0.040 | 3.35 | 153 | 12 |
| E4 | 0.040 | 3.34 | 190 | 37 |
| E5 | 0.040 | 3.34 | 165 | 25 |

In comparison with state-of-art representing compound C2, the inventive compounds enable longer lifetime and higher current efficiency.

**Table 4**

| **HBL** | **CIEy** | **Voltage, 10mA/cm² [V]** | **Ceff/CIEy, 10mA/cm² [cd/A]** | **LT97, 30mA/cm² [h]** |
|---|---|---|---|---|
| C3 | 0.047 | 3.36 | 158 | 11 |
| E3 | 0.052 | 3.35 | 170 | 25 |
| E4 | 0.048 | 3.26 | 186 | 41 |
| E5 | 0.047 | 3.26 | 163 | 29 |

In comparison with state-of-art representing compound C3, the inventive compounds enable longer lifetime and higher current efficiency.

**Table 5**

| **HBL** | **CIEy** | **Voltage, 10mA/cm² [V]** | **Ceff/CIEy, 10mA/cm² [cd/A]** | **LT97, 30mA/cm² [h]** |
|---|---|---|---|---|
| C4 | 0.046 | 3.35 | 163 | 6 |
| E6 | 0.046 | 3.34 | 198 | 17 |

In comparison with state-of-art representing compound C4, the inventive compound E6 enables longer lifetime and higher current efficiency.

The features disclosed in the foregoing description and in the dependent claims may, both separately and in any combination thereof, be material for realizing the aspects of the disclosure made in the independent claims, in diverse forms thereof.

## Claims

1. Compound of formula (I) 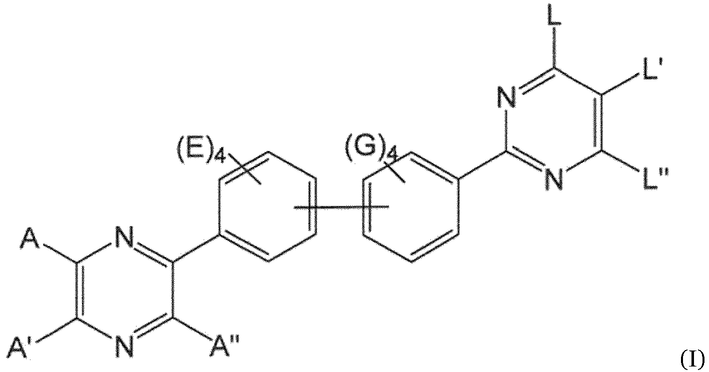 wherein
- A, A' and A" are independently selected from the group consisting of substituted or unsubstituted C₁ to C₂₀ alkyl, substituted or unsubstituted C₁ to C₂₀ alkenyl, substituted or unsubstituted C₆ to C₆₀ aryl, and substituted or unsubstituted C₂ to C₆₀ heteroaryl;
- L and L" are independently selected from the group consisting of substituted or unsubstituted C₆ to C₆₀ aryl and substituted or unsubstituted C₂ to C₆₀ heteroaryl, and L' is selected from the group consisting of H, D, C₁ to C₂₀ alkyl, substituted or unsubstituted C₁ to C₂₀ alkenyl, substituted or unsubstituted C₆ to C₆₀ aryl, and substituted or unsubstituted C₂ to C₆₀ heteroaryl;
or
- L is selected from the group consisting of substituted or unsubstituted C₆ to C₆₀ aryl and substituted and unsubstituted C₂ to C₆₀ heteroaryl, and L' and L" form together a condensed 6-membered aromatic ring,
wherein the condensed 6-membered aromatic ring
- is unsubstituted,
or
- is substituted with one or more substituent(s) independently selected from C₆ to C₅₆ aryl, provided that the total number of C-atoms of all substituents does not exceed 56,
or
- is condensed with a second 6-membered aromatic ring, wherein the condensed 6-membered aromatic ring and the second 6-membered aromatic ring are unsubstituted or at least one of the condensed 6-membered aromatic ring and the second 6-membered aromatic ring is substituted with one or more substituent(s) independently selected from C₆ to C₄₀ aryl provide that the total number of C-atoms of all substituents does not exceed 40;
- E is independently selected from the group consisting of H, D D, halogen, SiR^{a}R^{b}R^{c} (wherein R^{a}, R^{b} and R^{c} are independently selected from the group consisting of C₁ to C₆ alkyl and phenyl), C₁ to C₆ alkyl, C₁ to C₆ alkoxy, CN and P(=O)R^{d}R^{e} (wherein R^{d} and R^{e} are independently selected from the group consisting of C₁ to C₆ alkyl and phenyl);
- G is independently selected from the group consisting of H, D, halogen, SiR^{a}R^{b}R^{c} (wherein R^{a}, R^{b} and R^{c} are independently selected from the group consisting of C₁ to C₆ alkyl and phenyl), C₁ to C₆ alkyl, C₁ to C₆ alkoxy, CN and P(=O)R^{d}R^{e} (wherein R^{d} and R^{e} are independently selected from the group consisting of C₁ to C₆ alkyl and phenyl).

2. Compound according to claim 1, wherein A, A' and A" are independently selected from C₆ to C₆₀ aryl.

3. Compound according to claim 1 or 2, wherein A, A' and A" are each phenyl.

4. Compound according to any of the preceding claims, wherein L and L" are independently selected from substituted or unsubstituted C₆ to C₆₀ aryl and L' is H;
or
- L is selected from substituted or unsubstituted C₆ to C₆₀ aryl, and L' and L" form together a condensed 6-membered aromatic ring,
wherein the condensed 6-membered aromatic ring
- is unsubstituted,
or
- is condensed with a second 6-membered aromatic ring, wherein the condensed 6-membered aromatic ring and the second 6-membered aromatic ring are unsubstituted.

5. Compound according to any of the preceding claims, wherein L and L" are each phenyl and L' is H;
or
- L is phenyl and L' and L" form together an unsubstituted condensed 6-membered aromatic ring.

6. Compound according to any of the preceding claims, wherein E is H.

7. Compound according to any of the preceding claims, wherein G is H.

8. Semiconducting material comprising the compound of formula (I) according to any of the preceding claims.

9. Organic electronic device comprising a semiconducting layer, wherein the semiconducting layer comprises the semiconducting material according to claim 8.

10. Organic electronic device according to claim 9, wherein the semiconducting layer is a hole blocking layer and/or an electron transport layer and/or an electron injection layer.

11. Organic electronic device according to claim 9 or 10, wherein the semiconducting layer is a hole blocking layer.

12. Organic electronic device according to claim 11, wherein the organic electronic device further comprises an emission layer and an electron transport layer and the hole blocking layer is arranged between the emission layer and the electron transport layer.

13. Organic electronic device according to claim 12, wherein the organic electronic device is an organic light emitting diode.

14. Display device comprising the organic electronic device according to claim 13.

15. Process for preparing the organic electronic device according to claim 12 or 13, wherein the process comprises a step of depositing the compound according to any of the claims 1 to 8 on a solid support.

## Patentansprüche

1. Verbindung der Formel (I) 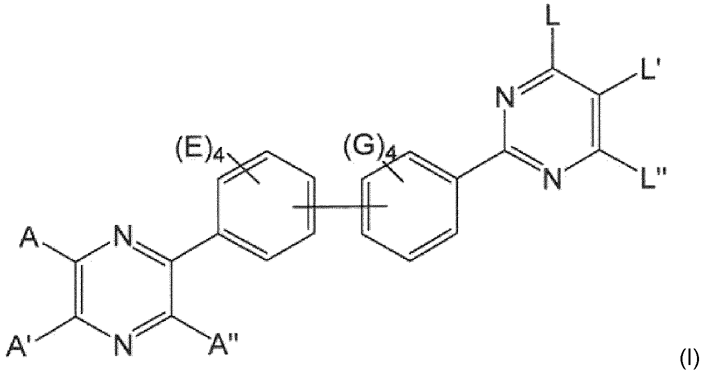 wobei
- A, A' und A" unabhängig ausgewählt sind aus der Gruppe bestehend aus substituiertem oder unsubstituiertem C₁ - bis C₂₀ -Alkyl, substituiertem oder unsubstituiertem C₁ - bis C₂₀ -Alkenyl, substituiertem oder unsubstituiertem C₆ - bis C₆₀ -Aryl und substituiertem oder unsubstituiertem C₂ - bis C₆₀ -Heteroaryl;
- L und L" unabhängig ausgewählt sind aus der Gruppe bestehend aus substituiertem oder unsubstituiertem C₆ - bis C₆₀ -Aryl und substituiertem oder unsubstituiertem C₂ - bis C₆₀ -Heteroaryl und L' ausgewählt ist aus der Gruppe bestehend aus H, D, C₁ - bis C₂₀ -Alkyl, substituiertem oder unsubstituiertem C₁ - bis C₂₀ -Alkenyl, substituiertem oder unsubstituiertem C₆ - bis C₆₀ -Aryl und substituiertem oder unsubstituiertem C₂ - bis C₆₀ -Heteroaryl;
oder
- L ausgewählt ist aus der Gruppe bestehend aus substituiertem oder unsubstituiertem C₆ - bis C₆₀ -Aryl und substituiertem und unsubstituiertem C₂ - bis C₆₀ -Heteroaryl und L' und L" zusammen einen kondensierten 6-gliedrigen aromatischen Ring bilden,
wobei der kondensierte 6-gliedrige aromatische Ring
- unsubstituiert ist,
oder
- mit einem oder mehreren Substituenten substituiert ist, die unabhängig ausgewählt sind aus C₆ - bis C₅₆ -Aryl, vorausgesetzt, dass die Gesamtzahl der C-Atome aller Substituenten 56 nicht überschreitet,
oder
- mit einem zweiten 6-gliedrigen aromatischen Ring kondensiert ist, wobei der kondensierte 6-gliedrige aromatische Ring und der zweite 6-gliedrige aromatische Ring unsubstituiert sind oder mindestens einer von dem kondensierten 6-gliedrigen aromatischen Ring und dem zweiten 6-gliedrigen aromatischen Ring mit einem oder mehreren Substituenten substituiert ist, die unabhängig ausgewählt sind aus C₆ - bis C₄₀ -Aryl, vorausgesetzt, dass die Gesamtzahl der C-Atome aller Substituenten 40 nicht überschreitet;
- E unabhängig ausgewählt ist aus der Gruppe bestehend aus H, D, D, Halogen, SiR^{a}R^{b}R^{c} (wobei R^{a}, R^{b} und R^{c} unabhängig ausgewählt sind aus der Gruppe bestehend aus C₁ - bis C₆ -Alkyl und Phenyl), C₁ - bis C₆ -Alkyl, C₁ - bis C₆ -Alkoxy, CN und P(=O)R^{d}R^{e} (wobei R^{d} und R^{e} unabhängig ausgewählt sind aus der Gruppe bestehend aus C₁ - bis C₆ -Alkyl und Phenyl);
- G unabhängig ausgewählt ist aus der Gruppe bestehend aus H, D, Halogen, SiR^{a}R^{b}R^{c} (wobei R^{a}, R^{b} und R^{c} unabhängig ausgewählt sind aus der Gruppe bestehend aus C₁ - bis C₆ -Alkyl und Phenyl), C₁ - bis C₆ -Alkyl, C₁ - bis C₆ -Alkoxy, CN und P(=O)R^{d}R^{e} (wobei R^{d} und R^{e} unabhängig ausgewählt sind aus der Gruppe bestehend aus C₁ - bis C₆ -Alkyl und Phenyl).

2. Verbindung nach Anspruch 1, wobei A, A' und A" unabhängig ausgewählt sind aus C₆ - bis C₆₀ -Aryl.

3. Verbindung nach Anspruch 1 oder 2, wobei A, A' und A" jeweils Phenyl sind.

4. Verbindung nach einem der vorhergehenden Ansprüche, wobei L und L" unabhängig ausgewählt sind aus substituiertem oder unsubstituiertem C₆ - bis C₆₀ -Aryl und L' H ist; oder
- L ausgewählt ist aus substituiertem oder unsubstituiertem C₆ - bis C₆₀ -Aryl und L' und L" zusammen einen kondensierten 6-gliedrigen aromatischen Ring bilden,
wobei der kondensierte 6-gliedrige aromatische Ring
- unsubstituiert ist,
oder
- mit einem zweiten 6-gliedrigen aromatischen Ring kondensiert ist, wobei der kondensierte 6-gliedrige aromatische Ring und der zweite 6-gliedrige aromatische Ring unsubstituiert sind.

5. Verbindung nach einem der vorhergehenden Ansprüche, wobei L und L" jeweils Phenyl sind und L' H ist;
oder
- L Phenyl ist und L' und L" zusammen einen unsubstituierten kondensierten 6-gliedrigen aromatischen Ring bilden.

6. Verbindung nach einem der vorhergehenden Ansprüche, wobei E H ist.

7. Verbindung nach einem der vorhergehenden Ansprüche, wobei G H ist.

8. Halbleitendes Material, umfassend die Verbindung der Formel (I) nach einem der vorhergehenden Ansprüche.

9. Organische elektronische Vorrichtung, umfassend eine halbleitende Schicht, wobei die halbleitende Schicht das halbleitende Material nach Anspruch 8 umfasst.

10. Organische elektronische Vorrichtung nach Anspruch 9, wobei die halbleitende Schicht eine Lochblockierschicht und/oder eine Elektronentransportschicht und/oder eine Elektroneninjektionsschicht ist.

11. Organische elektronische Vorrichtung nach Anspruch 9 oder 10, wobei die halbleitende Schicht eine Lochblockierschicht ist.

12. Organische elektronische Vorrichtung nach Anspruch 11, wobei die organische elektronische Vorrichtung ferner eine Emissionsschicht und eine Elektronentransportschicht umfasst und die Lochblockierschicht zwischen der Emissionsschicht und der Elektronentransportschicht angeordnet ist.

13. Organische elektronische Vorrichtung nach Anspruch 12, wobei die organische elektronische Vorrichtung eine organische Leuchtdiode ist.

14. Anzeigevorrichtung, umfassend die organische elektronische Vorrichtung nach Anspruch 13.

15. Verfahren zur Herstellung der organischen elektronischen Vorrichtung nach Anspruch 12 oder 13, wobei das Verfahren einen Schritt des Abscheidens der Verbindung nach einem der Ansprüche 1 bis 8 auf einem festen Träger umfasst.

## Revendications

1. Composé de formule (I) 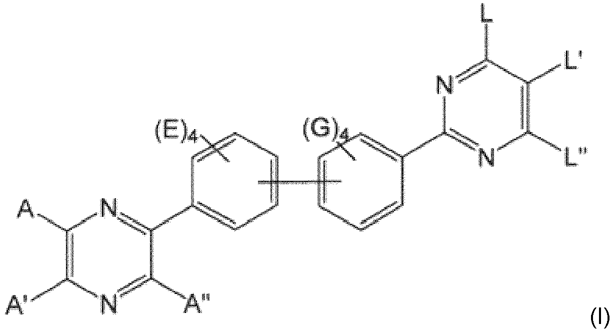 dans laquelle
- A, A' et A" sont choisis indépendamment dans le groupe constitué par les groupes alkyle en C₁ à C₂₀ substitués ou non substitués, les groupes alcényle en C₁ à C₂₀ substitués ou non substitués, les groupes aryle en C₆ à C₆₀ substitués ou non substitués et les groupes hétéroaryle en C₂ à C₆₀ substitués ou non substitués ;
- L et L" sont choisis indépendamment dans le groupe constitué par les groupes aryle en C₆ à C₆₀ substitués ou non substitués et les groupes hétéroaryle en C₂ à C₆₀ substitués ou non substitués, et le groupe L' est choisi dans le groupe constitué par H, D, un groupe alkyle en C₁ à C₂₀, un groupe alcényle en C₁ à C₂₀ substitué ou non substitué, un groupe aryle en C₆ à C₆₀ substitué ou non substitué, et un groupe hétéroaryle en C₂ à C₆₀ substitué ou non substitué ;
ou
- L est choisi dans le groupe constitué par les groupes aryle en C₆ à C₆₀ substitués ou non substitués et les groupes hétéroaryle en C₂ à C₆₀ substitués et non substitués, et L' et L" forment ensemble un cycle aromatique condensé à 6 chaînons,
dans laquelle le cycle aromatique condensé à 6 chaînons
- est non substitué,
ou
- est substitué par un ou plusieurs substituants choisis indépendamment parmi les groupes aryle en C₆ à C₅₆, à condition que le nombre total d'atomes de carbone de tous les substituants ne dépasse pas 56,
ou
- est condensé avec un deuxième cycle aromatique à 6 chaînons, dans laquelle le cycle aromatique condensé à 6 chaînons et le deuxième cycle aromatique à 6 chaînons ne sont pas substitués ou au moins l'un des cycles aromatiques condensés à 6 chaînons et le deuxième cycle aromatique à 6 chaînons est substitué par un ou plusieurs substituants choisis indépendamment parmi les groupes aryles en C₆ à C₄₀, à condition que le nombre total d'atomes de carbone de tous les substituants ne dépasse pas 40 ;
- E est indépendamment choisi dans le groupe constitué par H, D D, un halogène, un groupe SiR^{a}R^{b}R^{c} (dans lequel les radicaux R^{a}, R^{b} et R^{c} sont indépendamment choisis dans le groupe constitué par les groupes alkyle en C₁ à C₆ et phényle), un groupe alkyle en C₁ à C₆, un groupe alcoxy en C₄ à C₆, CN et P(=O)R^{d}R^{e} (dans lequel les radicaux R^{d} et R^{e} sont choisis indépendamment dans le groupe constitué par les groupes alkyle en C₁ à C₆ et phényle) ;
- G est choisi indépendamment dans le groupe constitué par H, D, un halogène, un groupe SiR^{a}R^{b}R^{c} (dans lequel les radicaux R^{a}, R^{b} et R^{c} sont choisis indépendamment parmi le groupe constitué par les groupes alkyle en C₁ à C₆ et phényle), un groupe alkyle en C₁ à C₆, un groupe alcoxy en C₁ à C₆, CN et P(=O)R^{d}R^{e} (dans lequel les radicaux R^{d} et R^{e} sont choisis indépendamment parmi le groupe constitué par les groupes alkyle en C₁ à C₆ et phényle).

2. Composé selon la revendication 1, dans lequel A, A' et A" sont choisis indépendamment parmi les groupes aryle en C₆ à C₆₀.

3. Composé selon la revendication 1 ou la revendication 2, dans lequel A, A' et A" représentent chacun un groupe phényle.

4. Composé selon l'une quelconque des revendications précédentes, dans lequel L et L" sont choisis indépendamment parmi les groupes aryle en C₆ à C₆₀ substitués ou non substitués et L' représente H ; ou
- L est choisi parmi les groupes aryle en C₆ à C₆₀ substitués ou non substitués, et L' et L" forment ensemble un cycle aromatique condensé à 6 chaînons,
dans lequel le cycle aromatique condensé à 6 chaînons
- est non substitué,
ou
- est condensé avec un deuxième cycle aromatique à 6 chaînons, dans lequel le cycle aromatique condensé à 6 chaînons et le deuxième cycle aromatique à 6 chaînons sont non substitués.

5. Composé selon l'une quelconque des revendications précédentes, dans lequel L et L" représentent chacun un groupe phényle et L' représente H ;
ou
- L est un groupe phényle et L' et L" forment ensemble un cycle aromatique condensé à 6 chaînons non substitué.

6. Composé selon l'une quelconque des revendications précédentes, dans lequel E représente H.

7. Composé selon l'une quelconque des revendications précédentes, dans lequel G représente H.

8. Matériau semi-conducteur comprenant le composé de formule (1) selon l'une quelconque des revendications précédentes.

9. Dispositif électronique organique comprenant une couche semi-conductrice, dans lequel la couche semi-conductrice comprend le matériau semi-conducteur selon la revendication 8.

10. Dispositif électronique organique selon la revendication 9, dans lequel la couche semi-conductrice est une couche bloquant les trous et/ou une couche de transport d'électrons et/ou une couche d'injection d'électrons.

11. Dispositif électronique organique selon la revendication 9 ou la revendication 10, dans lequel la couche semi-conductrice est une couche bloquant les trous.

12. Dispositif électronique organique selon la revendication 11, dans lequel le dispositif électronique organique comprend en outre une couche d'émission et une couche de transport d'électrons et la couche bloquant les trous est disposée entre la couche d'émission et la couche de transport d'électrons.

13. Dispositif électronique organique selon la revendication 12, dans lequel le dispositif électronique organique est une diode électroluminescente organique.

14. Dispositif d'affichage comprenant le dispositif électronique organique selon la revendication 13.

15. Procédé de fabrication du dispositif électronique organique selon la revendication 12 ou la revendication 13, dans lequel le procédé comprend une étape consistant à déposer le composé selon l'une quelconque des revendications 1 à 8 sur un support solide.
